# EUROPEAN PATENT SPECIFICATION

(11) **EP 3 397 242 B1**
(45) Date of publication and mention of the grant of the patent: **03.02.2021**
(21) Application number: 16829129.2
(22) Date of filing: 29.12.2016
(51) Int. Cl.: A61K 9/00, A61K 9/107, A61K 47/44, A61K 31/00, A23L 33/00

(54) **LIPOPHILIC FORMULATIONS**
LIPOPHILE FORMULIERUNGEN
FORMULATIONS LIPOPHILES

(30) Priority: 29.12.2015 EP 15003678
(43) Date of publication of application: 07.11.2018
(73) Proprietor: Noivita S.R.L.S., 28100 Novara (NO) (IT)
(72) Inventor: MOLINARI, Claudio Giuseppe, 28100 Novara (NO) (IT); UBERTI, Francesca, 28838 Stresa (Verbano Cusio Ossola) (IT)
(74) Representative: Biggi, Cristina
(86) International application number: PCT/IB2016/058076
(87) International publication number: WO 2017/115316

(56) References cited:
- WO-A1-2009/067734
- GB-A- 2 488 788

## Description

### Technical field

The present invention refers to a composition comprising a combination of castor and linseed oils as delivery system for lipidic substances. Moreover, the present invention refers to a process for producing the composition as nanoemulsion. In particular, the composition, eventually as nanoemulsion, is edible and is useful as a medicament or as dietary supplements.

### Background art

Lipids and lipophilic excipients can have significant and beneficial effects on the absorption and exposure of co-administered lipophilic drugs. There are three primary mechanisms by which lipids and lipophilic excipients affect drug absorption, bioavailability and disposition after oral administration. These are the alteration of the composition and character of the intestinal milieu, the recruitment of intestinal lymphatic drug transport, and the interaction with enterocyte-based transport processes. Lipid-based drug delivery (LBDD) systems have gained much importance in recent years due to their ability to improve the solubility and bioavailability of drugs with poor water solubility. Some of the drugs that are successfully marketed as lipid based formulations include efavirenz, saquinavir, ritonavir, clofazamine.

Refined oils are among the most commonly used lipid vehicles, for both pharmaceutical products and food supplements. As stated above, a disadvantage associated with refined oils as well as other lipid vehicles is poor oxidative stability. Unsaturated lipids are generally more prone to direct oxidation via rearrangement or loss of double bonds and in some instances the degradation of lipid tails, possibly leading to membrane reorganization, changes in the packing of membrane components, lateral fluidity, domain reorganization or molecular redistributions, increased permeability, and membrane phase behavior. Moreover, some of these membrane reorganizations may also influence the conformations and thus the functions of selected membrane proteins, which in turn characterize the pathological condition.

It is known that the oxidant-antioxidant balance is critical for immune cell function because it maintains the integrity and functionality of the cell membrane, cellular proteins and nucleic acids. Moreover, this balance is important in controlling signal transduction and gene expression.

Some plant- derived oils are used extensively in nutrition, cosmetics and lipochemistry.

Refined vegetable oils are also used as solvents and co-solvent for pharmaceutical formulations as well as food supplements and vitamins; these include medium chain triglycerides, oleic acid, a-tocopherol, corn oil, peanut oil, sesame oil, sunflower oil, olive oil.

Although traditionally pharmacopoeias indicated refined oils as excipient for active principles characterized by low water solubility, more recently non-refined vegetable oils have become more and more interesting as excipients for both the pharmaceutical industry and the food supplement industry, due to their high nutritional values and antioxidant characteristics.

Particularly interesting vegetable oils are extra virgin olive oil, borrago officinalis oil, castor oil, linseed oil or flaxseed oil. Castor oil was used in Egypt as a lamp oil about 6000 years ago. The oil is typically composed of more than 94% triacylglycerols, the remaining being monoglycerides (3.4%), and phospholipids (0.6%). Castor oil is the only commercial source of ricinoleate. Its specific fatty acid composition is typically: 1% 18:0, 3% 18:1, 3% 18:2, about 90% hydroxylated 18:1 (ricinoleic acid) and 1% dihydroxylated 18:2. About 70% of castor oil is tri-ricinolein (tri-ricinoleoylglycerol). Moreover, 0.5% of the lipid content is composed of a tetra-acylglycerol containing four ricinoleic acid molecules (12-ricinoleoylricinoleoyl) di-ricinoleoylglycerol), 95% of the 12-ricinoleoylricinoleoyl chain being identified at the sn-2 position of the glycerol backbone. Castor oil components and many of their derivatives are stabilized by the hydroxyl groups, which are beta to the double bond. These hydroxyl groups protect the double bond by preventing the formation of peroxides. As a result, castor oil is approximately four times more stable than olive oil. The oil is more dense and viscous than most other vegetable oils and completely miscible with ethanol. Castor oil is largely used in lipochemistry (drying oils, paints, ink, soaps, lubricants, varnish), cosmetics (non-comedogens and emollients) and food industry. Castor oil has been successfully utilized in cosmetic products like skin lotions, shampoos, lipsticks, as well as excipient for pharmaceutical formulations. For instance, it has been shown to slow down absorption of intravenously and orally administered drugs, to improve the quality of the tear film in the eye when administered topically to the eye, improving dry eye symptoms. The immunosuppressant drug, tacrolimus, has been administered as emulsion with castor oil, showing reduced red blood cell toxicity. Castor oil formulations have been shown to protect drugs from the acidic milieu of the stomach, with a constant and prolonged absorption of the drug.

Flaxseed oil, also called linseed oil, is the oil obtained from the dried, ripened seeds of the flax plant (*Linum usitatissimum*). Linseed oil is a drying oil, meaning it can polymerize into a solid form. Due to its polymer-forming properties, linseed oil can be used on its own or blended with combinations of other oils, resins or solvents as an impregnator, drying oil finish or varnish in wood finishing, as a pigment binder in oil paints, as a plasticizer and hardener in putty, and in the manufacture of linoleum. Flaxseed is rich in Omega-3 fatty acids. It is promoted for heart, skin, and immune system health. Linseed oil, typically derived from *Linum usitatissimum,* is typically composed of 57% ω-3, 16% ω-6, 28% monounsaturated fatty acids, and only 9% unsaturated fatty acids, and the ω-3-to-ω-6 ratio of 1:3 is considered close to ideal. Linseed oil is considered to be a natural anti-inflammatory agent due its potential role in increasing non-inflammatory mediators, such as PGE1 and TXA1. Although there are no FDA approved medical uses for flaxseed oil, it has been used as laxative for constipation, for weight loss, and to prevent breast cancer and prostate cancer. Flaxseed oil is also applied to the skin to sooth irritations or soften roughness. As a source of polyunsaturated fatty acids such as alpha-linolenic acid, it has been used to decrease inflammation and thought to be useful for rheumatoid arthritis and other inflammatory (swelling) diseases.

WO2009/067734 discloses an oil-in-water nanoemulsion for the delivery of active components comprising an oil phase, a non-ionic surfactant and an aqueous phase. GB2488788 discloses an edible composition comprising polyoxyethylene glycolated castor oil and linseed oil in a volumetric ratio of 41:59.

Vitamin D (vitD), also known as calciferol, comprises a group of fat-soluble seco-sterols. The two major forms are vitamin D2 and vitamin D3. Vitamin D2 (ergocalciferol) is a plant derived form of Vitamin D, whereas vitamin D3 (cholecalciferol) is synthesized in the skin of humans from 7-dehydrocholesterol and is also consumed in the diet via the intake of animal-based foods. Vitamin D, in either the D2 or D3 form, is considered biologically inactive until it undergoes two enzymatic hydroxylation reactions. The first takes place in the liver, mediated by the 25-hydroxylase (most likely cytochrome P450 2R1 [CYP2R1]) which forms 25-hydroxyvitamin D (hereafter referred to as 25OHD). The second reaction takes place in the kidney, mediated by 1α-hydroxylase (CYP27B1), which converts 25OHD to the biologically active hormone, calcitriol (1,25-dihydroxyvitamin D). The 1α-hydroxylase gene is also expressed in several extra-renal tissues, but its contribution to calcitriol formation in these tissues is unknown. 25OHD, the precursor of calcitriol, is the major circulating form of vitamin D; it circulates bound to a specific plasma carrier protein, vitamin D binding protein (DBP). DBP also transports vitamin D and calcitriol. Following its synthesis in the kidney, calcitriol binds to DBP to be transported to target organs. The biological actions of calcitriol, involve regulation of gene expression at the transcriptional level, and are mediated through binding to a vitamin D receptor (VDR), located primarily in the nuclei of target cells. Additional hydroxylation reactions, such as that mediated by CYP24A1, result in more polar metabolites with greatly reduced or no apparent biological activity. The classical actions of vitamin D take the form of the regulation of serum calcium and phosphate homeostasis and, in turn, the development and maintenance of bone health. Non-classical functions have been linked to VDRs present in tissues not involved with calcium and phosphate homeostasis, where calcitriol may play a more general role. Furthermore, vitamin D-responsive elements (VDREs), considered the hallmark of vitamin D action, are present in a large number of human genes involved in the regulation of cell proliferation, cell differentiation, and apoptosis. Calcitriol has been shown to exert immunomodulatory and anti-proliferative effects through autocrine and paracrine pathways. Furthermore, calcitriol has been shown to play a potential role in preventive or therapeutic action in cancer and chronic conditions such as autoimmune conditions (including type 1 diabetes), cardiovascular disease, and infections.

Binding its receptor, vitD causes a variety of downstream events, including a protective role against oxidative stress, regulation of autophagic pathways, and the interplay between apoptosis and survival pathways.

The dietary sources of vitamin D include food and dietary supplements. There are a few naturally occurring food sources of vitamin D, including fatty fish, fish liver oil, and egg yolk. Some foods are, however, fortified with vitamin D: after vitamin D was recognized as important for the prevention of rickets in the 1920s, vitamin D fortification of some foods was initiated. Nowadays, dietary supplements containing vitamin D have become more common and the form of vitamin D used in such products can be either vitamin D2 or vitamin D3. Vitamin D2 (VD2) was the first form of Vitamin D produced industrially starting from the 1920s and used to manufacture the first Vitamin D based products used to treat rickets.

Although international pharmacopoeias have historically recognized the two forms of Vitamin D as equivalent, and VD2 has proven efficient in eradicating rickets upon food supplementation, in recent years a growing body of evidence suggests a greater bioefficacy of vitamin D3 (VD3). New bioassays, like measurement of serum 25-hydroxyvitamin D [25(OH)D] as marker of vitamin D levels, have shown that VD3 is on average 2-3 folds more bioactive than VD2: VD3 is more effective than VD2 at raising serum 25(OH)D concentrations, and has greater capacity to maintain higher 25(OH)D concentrations over time.

In recent years, there has been an increased interest in optimizing supplementation strategies for vitamin D in populations at risk for vitamin D insufficiency and chronic disease, such as the elderly, obese individuals, and chronic kidney disease.

Several studies have reported differences in the bioavailability of vitamin D supplements in some populations. Decreased bioavailability may be due to altered absorption of vitamin D from the small intestine or it may be due to altered metabolism of vitamin D in the body. Intestinal malabsorption disorders, like for instance cystic fibrosis, may cause a decrease in vitamin D absorption due to a decreased ability to absorb lipids. Obesity has also been found to be associated with decreased 25(OH)D levels and may reflect the larger body volume of obese individuals or the sequestration of vitamin D in excess adipose tissue.

Vehicles such as oils, powders, and ethanol can be used in vitamin D supplements; however, the vehicle may have an impact on the bioavailability of vitamin D supplements: the mechanism of vitamin D absorption from the gastrointestinal tract is similar to the absorption of other lipids. In the intestine, vitamin D is associated with micelles and is taken up with other lipids by passive diffusion into the enterocytes. The presence of fat in the duodenum stimulates the release of bile acids to facilitate lipid absorption. Vitamin D may require other lipids to stimulate the release of bile acids and with which to associate in micelles to facilitate its absorption by the intestinal mucosa. Therefore, vitamin D may exhibit differential efficacy in absorption when solubilized in oil, lactose powders, cellulose powders or ethanol.

The optimization of VD3 formulation has been difficult since it is known to be highly unstable. The major cause of this instability is its degradation particularly in the presence of oxygen, light and humidity. This lack of stability may often be detected as a drop in the level of VD3 in a formulation. It has therefore been difficult to develop and market stable formulations of cholecalciferol. Liquid formulations of VD3 have a better bioavailability profile compared to dry formulations, eg are easily absorbed. VitaminD3 can be dissolved in Ethanol, which is not a solvent of first choice since adverse CNS and neurotoxic effects have been reported, especially after repeated use.

Some attempts have been made to provide liquid compositions of vitamin D3, for instance in WO2012117236, WO2003059358, WO2008031188, US20030191093, US20090196862, incorporated herein in their entirety.

There thus remains however need for vitamin D preparations that can deliver therapeutic doses of vitamin D in a pharmaceutically acceptable form for the rapid repletion and maintenance of vitamin D levels in people with vitamin D deficiency or insufficiency. Such preparations can further be used to treat or prevent of a variety of health conditions that are affected by low levels of vitamin D.

Over a century ago, Acetyl salicylic acid, also known as Aspirin, founded a new class of drugs: the nonsteroidal anti-inflammatory drugs (NSAIDs), due to its antipyretic and analgesic properties. Afterwards, Aspirin use was extended to the treatment of rheumatism and to the prevention of cardiovascular diseases, diabetes and obesity. Acetylsalicylic acid (ASA) is able to exert anti-inflammatory effects through multiple mechanisms of action that may include generation of reactive oxygen species (ROS), increase of oxidative stress, mitochondrial dysfunction and induction of apoptosis. Unfortunately, the same mechanisms are also involved in its cytotoxic effects. Furthermore, ASA is considered a promising candidate for the prevention and treatment of some cancers. In particular it has been recently shown in several cancer cell lines both therapeutic and prophylactic role for this substance.

However, an important limitation to long-term and high dose treatment with Aspirin is its inherent toxicity, based on the generation of reactive oxygen species (ROS) and increase of oxidative stess. Many pathologies of stomach and liver are caused by oxydative injury, via ROS or Nitric oxide, and adverse effects observed in liver and stomach by long term pharmacological treatment with the same drugs (such as ASA) have been linked to increase of the oxidative stress condition.

Therefore, there is a need for a formulation of ASA, which helps balance its long term and high dose toxicity. Surprisingly, the present inventors have found that VD3 is able to decrease the ASA mediated oxidative stress, also in conditions of additional external oxidation, by counteracting activation of apoptosis pathways.

The present invention solves the needs reported above with new vegetable oil based formulations comprising linseed oil and castor oil that decrease the cellular production of reactive oxygen species, when compared to known lipidic formulations and hence decrease the solvent mediated oxidative stress. The oil-based composition of the invention is a lipid-based drug delivery system useful to supply an individual with lipidic substances difficult to formulate and therefore difficult to intake. Moreover, the present invention refers to a nanoemulsion method to prepare the mixed oils and methods to transform the liquid formulation in a powder or solid formulation, such as gelatin 20%, silica gel, spray-congealing/spray drying or adding maltodextrin.

In particular, the composition is useful to deliver lipidic vitamins, especially D3 vitamin. A preferred composition comprises besides the oils disclosed D3 vitamin and ASA.

### Brief description of drawings

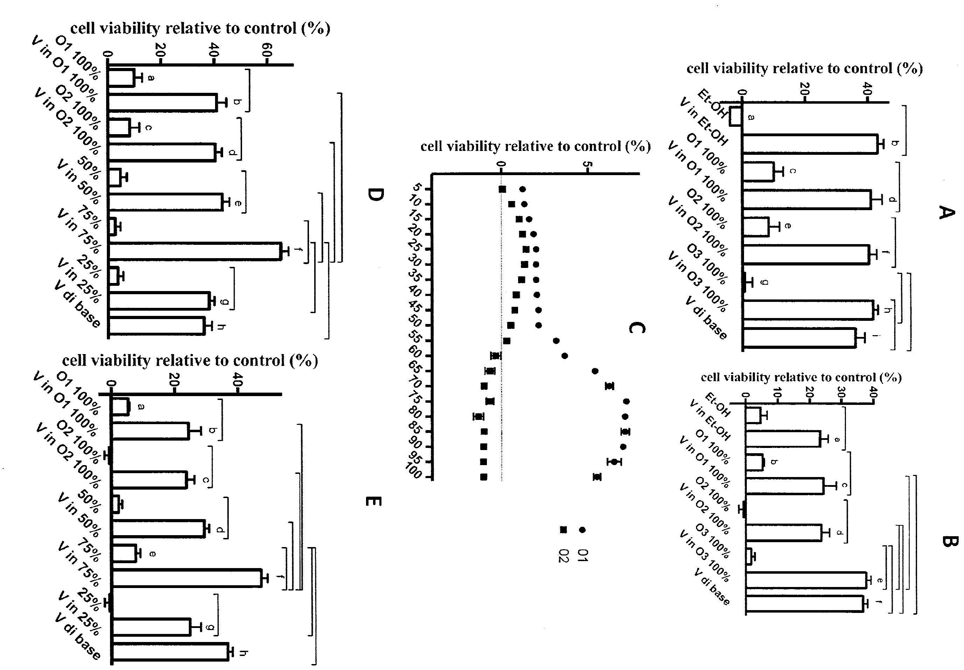

- Figure 1 shows cell viability of HuH-7 (A, C and D) and GTL-16 cells (B and E) upon treatment with vegetable oils and/or Vitamin D3, measured by MTT assay. In particular, Fig.1A and B show the effects of pure oils alone or in association with vitD₃ and the commercial vitD effects. Et-OH= ethanol; V in Et-OH = vitD₃ dissolved in 100% ethanol; O1 100%= linseed oil; V in O1 100%= vitD₃ dissolved in 100% linsed oil; O2 100% = castor oil; V in O2 100%= VitD₃ dissolved in 100% castor oil; O3 100%= olive oil 100%; V in O3 100%= vitD₃ dissolved in 100% olive oil; V di base= commercial vitD. Fig.1C shows different concentrations (range from 5% to 100%) of linseed and castor oils tested alone on MTT test of Huh-7 cells. Fig.1D and E show the effects of pure oils alone or in combination and in association with vitD₃ and of the commercial vitD. 50%= 50% 01+50% O2; V in 50%= vitD₃ dissolved in 50% solution; 75%= 75% O1+ 25% O2; V in 75%= vitD₃ dissolved in 75% solution; 25%= 25% O1+75% O2; V in 25%= vitD₃ dissolved in 25% solution; the other abbreviations are the same described before. All the data are expressed as a means±SD (%) normalized to control values (0 %) of 5 independent experiments.

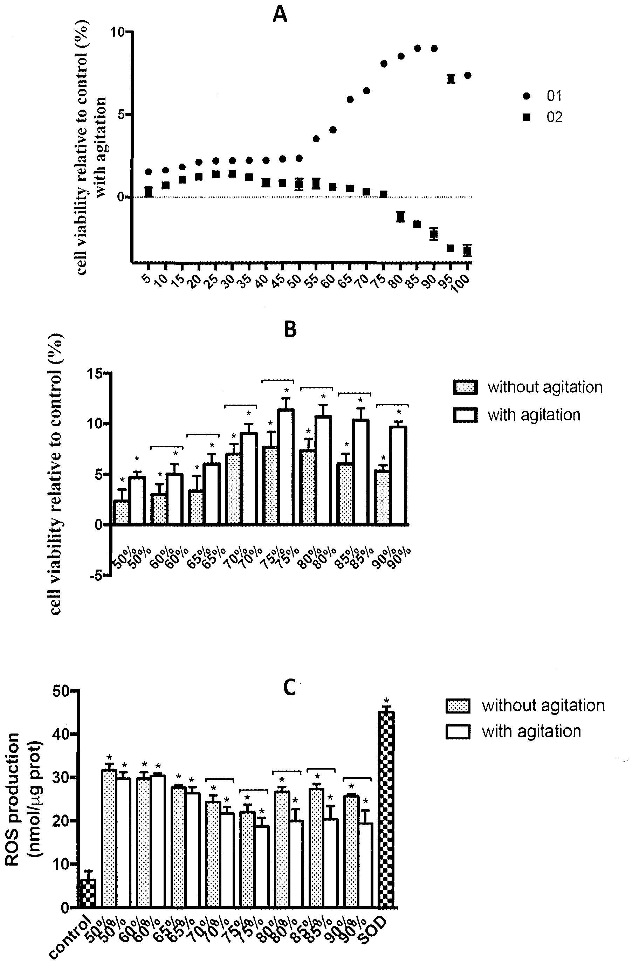

- Figure 2 shows cell viability (A and B) and ROS production (C) measured in GTL-16 cells. In particular, Fig.2A shows different concentrations (range from 5% to 100%) of nanoemulsion linseed and castor oils tested alone on MTT. O1 = linseed oil; O2 = castor oil; for linseed oil all dilutions are p<0.05 vs control; for castor oil from 15% to 90% p<0.05 vs control. These data were expressed as a means±SD (%) normalized to control values (0 %) of 5 independent experiments. Fig.2B shows the effect of combined oils on cell viability (MTT test). 50%= 50% O1+50% O2; 60%=60% O1+40% O2; 65%=65% O1+35% O2; 70%=70% O1+30% O2; 75%= 75% O1+25% O2; 80%=80% O1+20%O2; 85%=85% O1+15%O2; 90%=90% O1+10% O2. These data were expressed as a means±SD (%) normalized to control values (0 %) of 5 independent experiments. *p<0.05 vs control; the bars indicated p<0.05 between the nanoemulsion combined of oils and the same combination without emulsion. Fig.2C shows ROS production of combined oils. The abbreviations are the same reported in B. *p<0.05 vs control; the bars indicated p<0.05 between the nanoemulsion combined of oils and the same combination without emulsion. These data were expressed as a means±SD of nmol of cytochrome c reduced/µg of protein obtained in 5 independent experiments.

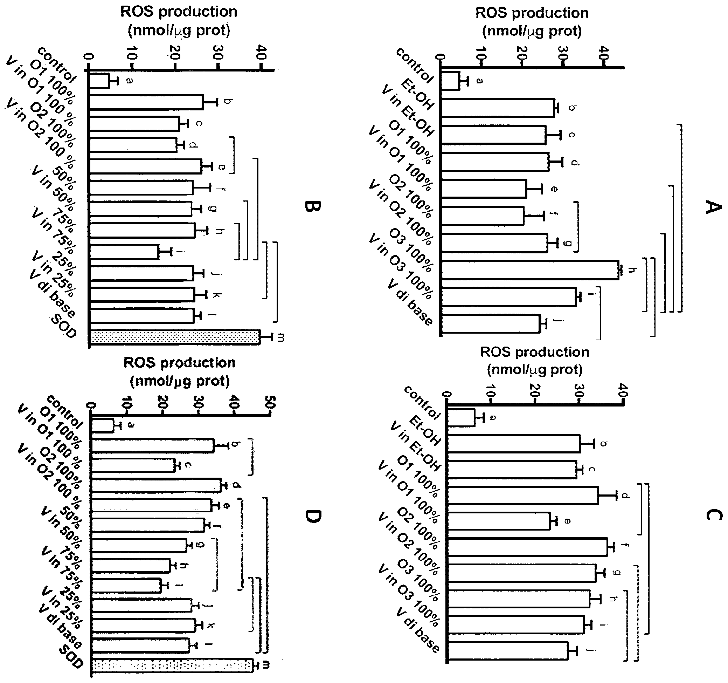

- Figure 3 shows ROS production measured in HuH-7 (A and B) and GTL-16 cells (C and D). In particular, Fig. 3A and C show the effects of pure oils alone or in association with VitD₃ and of the commercial vitD. The abbreviations are the same reported in Fig.1. Fig.3B and D show the effects of pure oils alone or in combination and in association with vitD₃ and of the commercial vitD. SOD=superoxide dismutase production; the other abbreviations are the same reported in Fig. 1. All the data are expressed as a means±SD of nmol of cytochrome c reduced/µg of protein obtained in 5 independent experiments.

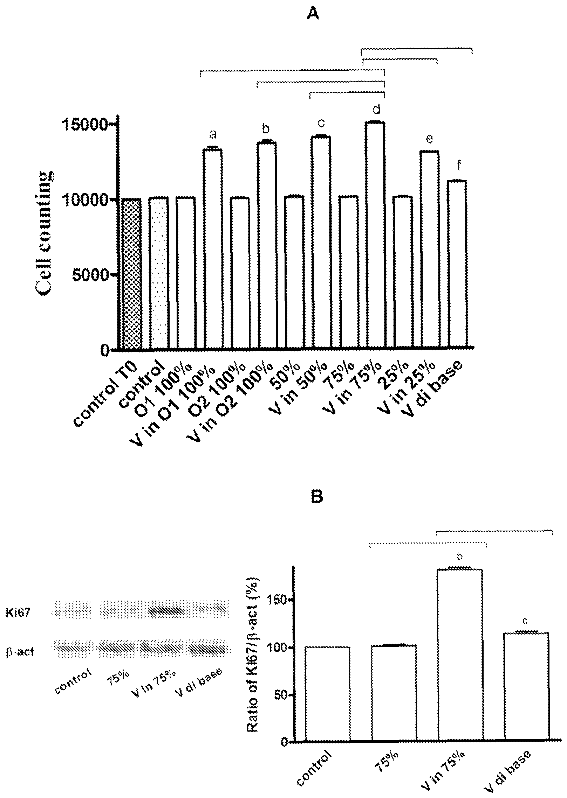

- Figure 4 shows cell proliferation of HuH7 cells. In particular, Fig.4A shows the cell counting measured by crystal violet staining after the stimulation of the same agents reported previously. The abbreviations are the same reported in Fig. 1. All the data are expressed as a means±SD of cell counted in 5 independent experiments. Fig.4B shows the Western blot (panel on the left) and densitometric analysis (panel on the right) of Ki67 in stimulated Huh-7 cells. The data are representative of 5 independent experiments. The abbreviations are the same used before.

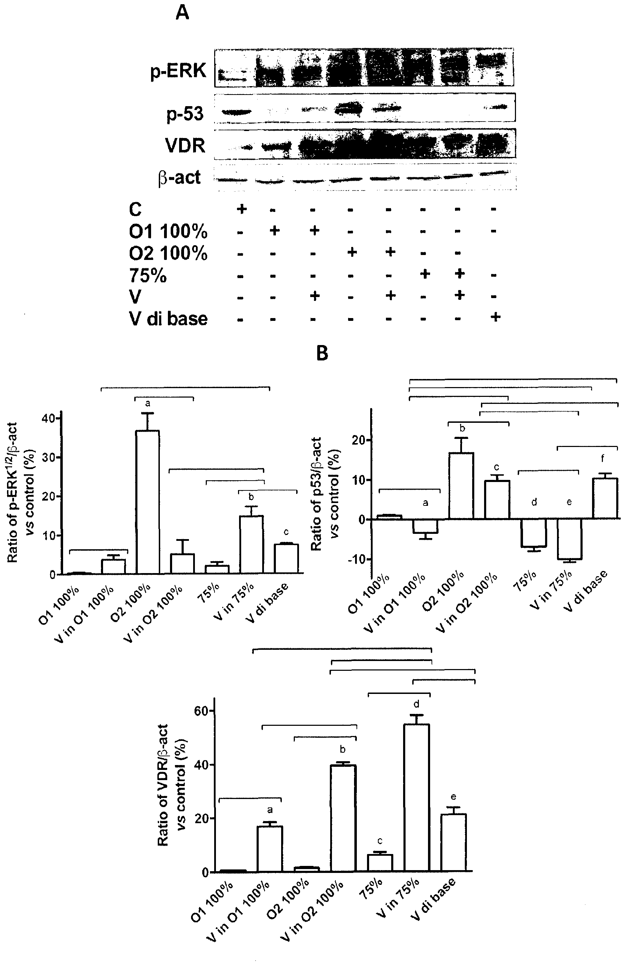

- Figure 5 shows Western blot (A) and densitometric analysis (B) of ERK/MAPK, p53 and VDR in HuH-7 cells. The abbreviations are the same reported in Fig. 1. The pictures are representative examples of 5 independent experiments, and densitometric analysis was expressed like a ratio of means±SD (%) of the same experiments.

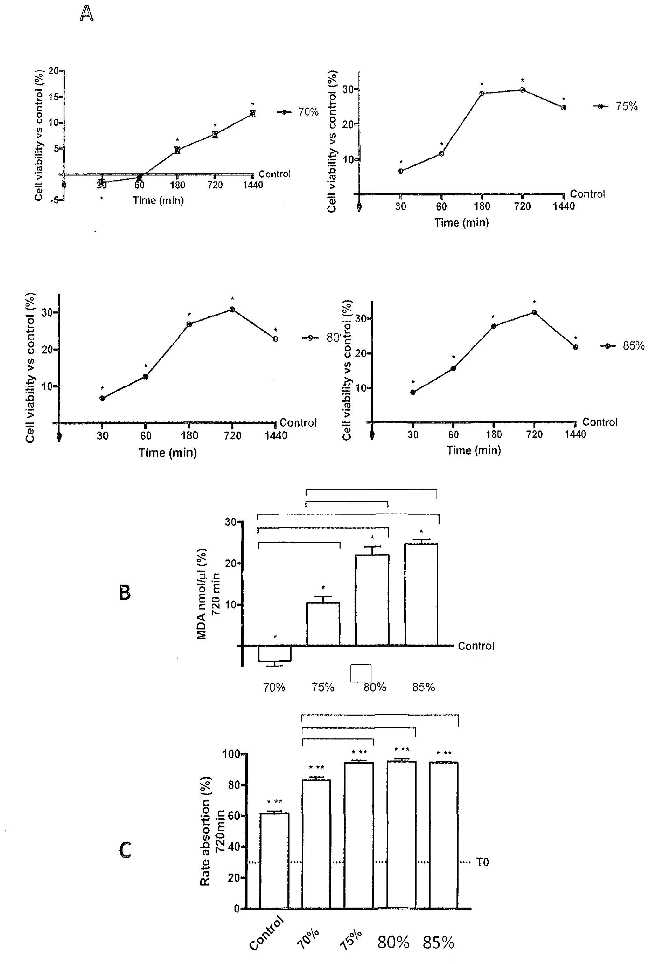

- Figure 6 shows cell viability (A), MDA produced (B) and rate of absorption measured in Caco-2 cells. Fig.6A shows different concentrations of combined nanoemulsion linseed and castor oils (range from 70% to 85%) during time (from 30 to 1440 min) on MTT. O1 = linseed oil; O2 = castor oil. 70%=70% O1+30% O2; 75%= 75% O1+ 25% O2; 80%=80% O1+20%O2; 85%=85% O1+15%O2. From 30min to 1440 min 75%, 80%, 85% are p<0.05 *vs* control; 70% is p<0.05 *vs* control after 180 min to 1440 min. The data are expressed as a means±SD (%) normalized to control values (0 %) of 5 independent experiments. Fig.6B shows MDA production of combined oils using peroxidation kit measured at 720 min. The abbreviations are the same used in A. The data are expressed as a means±SD (%) normalized to control values (0 %) of 5 independent experiments. *p<0.05 vs control; the bars indicated p<0.05 between the different combination of nanoemulsion combined oils. Fig.6C shows the absorption rate at 720 min in Caco-2 cell treated with different combination of nanoemulsion combined oils, calculated through the down volume present downstream of transwell. T0= volume present at the beginning of the stimulation. The other abbreviations are the same reported in A. *p<0.05 vs control; ** p<0.05 *vs* T0; the bars indicated p<0.05 between the different concentration of nanoemulsion combined oils. The data were expressed as a means±SD (%) obtained in 5 independent experiments.

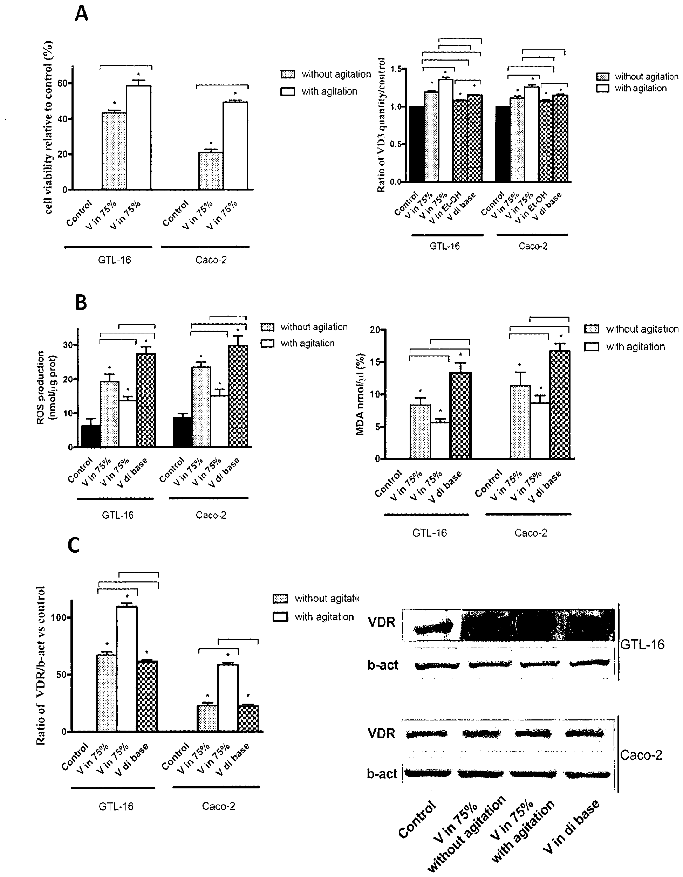

- Figure 7 shows the analysis of the effects of VD3 prepared in nanoemulsion 75% linseed oils/25% castor oils mixture compared to VD3 prepared in 75% linseed oils/25% castor oils mixture without nanoemulsion. Fig.7A shows cell viability on GTL-16 and Caco-2. Fig.7B shows the ratio of quantity of VD3 on intracellular level relative to control in GTL-16 and Caco-2. Fig.7C shows ROS produced in GTL and Caco-2 cells. Fig.7D shows MDA produced in GTL and Caco-2 cells measured by peroxidation kit. Fig.7E and F show respectively the densitometric analysis and Western blot of VDR receptor in GTL and Caco-2. The abbreviations are the same used before. Without agitation= without nanoemulsion; With agitation=with nanoemulsion. The data are expressed as a means±SD(%) of 5 independent experiments. * p<0.05 vs control; the bars indicated p<0.05 between with or without nanoemulsion.

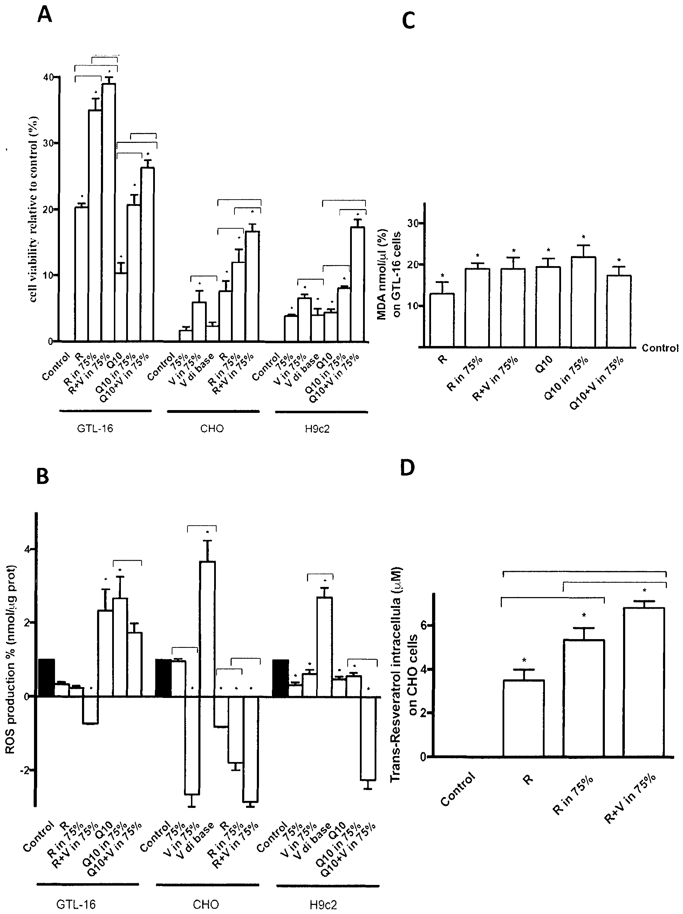

- Figure 8 shows the effect of nanoemulsion mixed oils on GTL-16, CHO and H9c2 cells. In particular, Fig.s8A and B show respectively the cell viability and the ROS production of GTL, CHO and H9c2 in presence of Resveratrol and Q10 solubilized with nanoemulsion mixed oils and with or without VD3. Fig.8C shows the MDA produced in GTL cells measured by peroxidation kit. Fig.8D shows the trans-resveratrol quantity obtained on intracellular fraction of CHO cells using HPLC system. R=Resveratrol; Q10=Q10 co-enzyme. The other abbreviations are the same used before. The data are expressed as a means±SD(*µ*M) of 5 independent experiments. * p<0.05 vs control; the bars indicated p<0.05 between the same agents differently formulated.

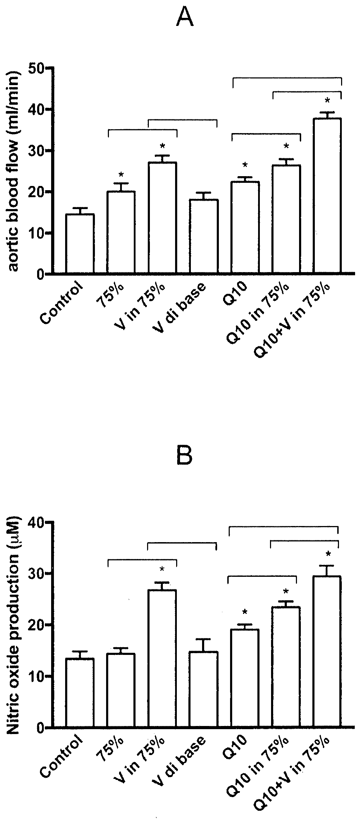

- Figure 9 shows the aortic blood flow (A) and nitric oxide (B) release on animal model. The abbreviations are the same used previously and the data are expressed as a means±SD (*µ*M) of 6 independent experiments. * p<0.05 vs control; the bars indicated *p*<0.05 between the same agents differently formulated.

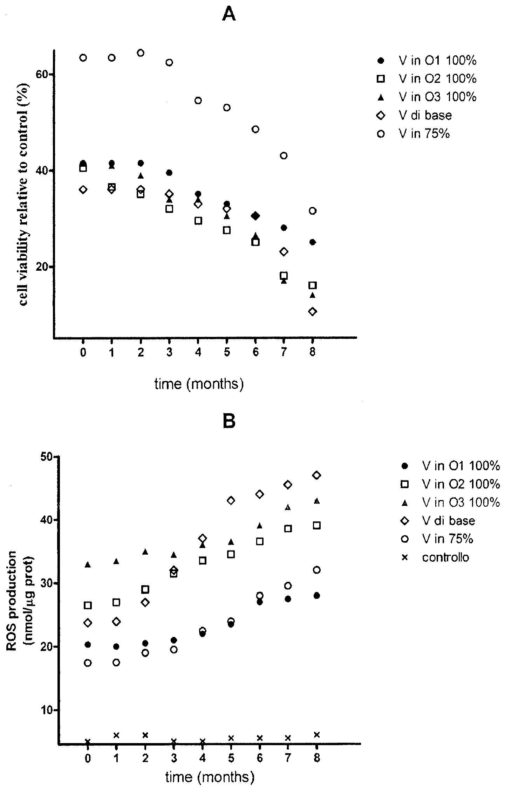

- Figure 10 shows the time-course study of cell viability and ROS production on HuH-7 cell lines. In particular, Fig.s10A and B show respectively a time-course of (A) cell viability and (B) ROS production in HuH-7 cells treated with different formulation of oils and vitamin D3 (vitamin D3 dissolved in 100% linseed oil, in 100% castor oil, in 100% olive oil, in 75%linseed oil plus 25% castor oil, and the commercial vitamin D). The tests are performed after the first time every month for 8 months. In A the results are expressed as a means±SD (%) of 3 technical replicates and during all time V in 100% O1, V in 100% O2, V in 100% O3, V in 75% were p<0.05 vs control values. V di base p<0.05 vs control values till to seven months; in B data are expressed as a means±SD of nmol of cytochrome c reduced/µg of protein obtained in 3 independent experiments. All data were p<0.05 vs control during all period.

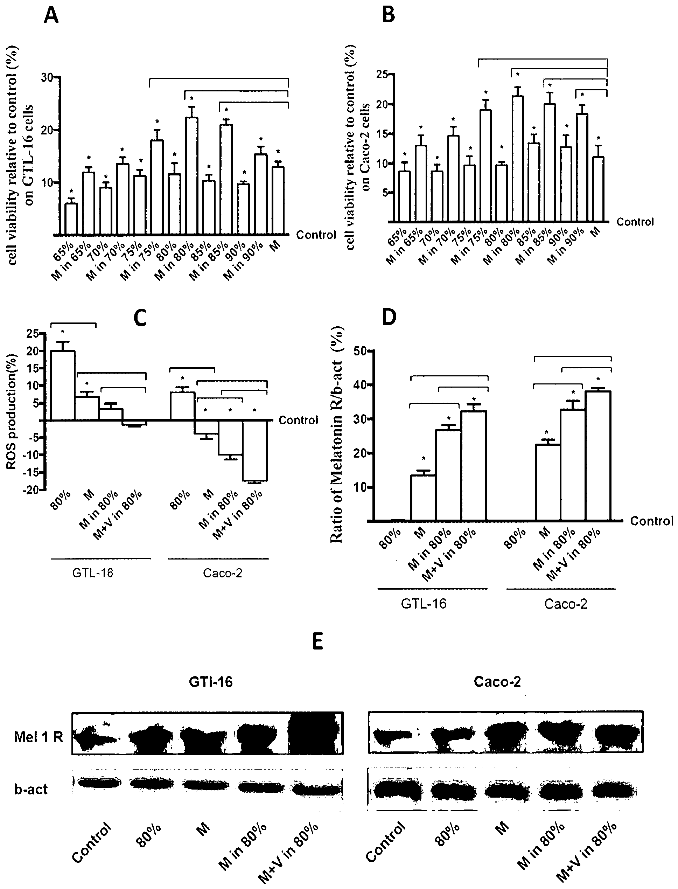

- Figure 11 shows the effect of melatonin on GTL-16 and Caco-2 cells. In particular, Fig.s11A and B show cell viability on GTL-16 and Caco-2 cell respectively, of Melatonin (M) prepared in different nanoemulsion mixed oils (range from 65% to 90%). Fig.11C shows the ROS production of GTL, and Caco-2 cells in presence of M with nanoemulsion 80% linseed/20%castor mixed oils, with or without VD3. Fig.11D and E show respectively the densimetric and the Western blot analysis of melatonin 1 receptor in GTL, and Caco-2 cells. The abbreviations are the same used previously. The data are expressed as a means±SD (%) of 5 independent experiments. * p<0.05 vs control; the bars indicated p<0.05 between M differently formulated.

### Detailed description of preferred embodiments of the invention

As used herein, a dietary (food) supplement is a substance intended to provide nutrients that may otherwise not be consumed in sufficient quantities. The FDA defines dietary supplements as products intended for ingestion that contains a "dietary ingredient" intended to add further nutritional value to (supplement) the diet. A "dietary ingredient" may be one, or any combination, of the following substances: a vitamin, a mineral, an herb or other botanical, an amino acid, a dietary substance for use by people to supplement the diet by increasing the total dietary intake, a concentrate, metabolite, constituent, or extract.

Dietary supplements may be found in many forms such as tablets, capsules, softgels, gelcaps, liquids, or powders. Some dietary supplements can help ensure that you get an adequate dietary intake of essential nutrients; others may help you reduce your risk of disease.

The European Food Safety Authority (EFSA) defines food supplements as follows: Food supplements are concentrated sources of nutrients or other substances with a nutritional or physiological effect, whose purpose is to supplement the normal diet. Food supplements are marketed 'in dose' form, for example as pills, tablets, capsules or liquids in measured doses etc. Supplements may be used to correct nutritional deficiencies or maintain an adequate intake of certain nutrients. However, in some cases excessive intake of vitamins and minerals may be harmful or cause unwanted side effects; therefore, maximum levels are necessary to ensure their safe use in food supplements.

As used herein, Botanicals and derived preparations made from plants, algae, fungi or lichens have become widely available on the EU market in the form of food supplements. Examples include ginkgo, garlic, St. John's Wort and ginseng. Such products are typically labeled as natural foods and a variety of claims are made regarding possible health benefits. They can be bought over the counter in pharmacies, supermarkets, specialist shops and via the Internet.

As used herein, the term nutraceutical is defined as, a food (or part of a food) that provides medical or health benefits, including the prevention and/or treatment of a disease. "it is a term commonly used in marketing, with no regulatory definition. The term was coined from "nutrition" and "pharmaceutical"

As used herein, Vitamin D3 (VD3) is cholecalciferol. Vitamin D (VD) is commonly used to indicate mixtures of cholecalciferol, ergocalciferol, and other metabolites which have bioactivity through interaction with the Vitamin D receptor, albeit at lower levels than cholecalciferol alone. In the context of the present invention, Vitamin D3 is measured in "International Units". In the context of the present invention, the conversion factor between International Units and grams is 0.025, eg the biological activity of 40IU (International Unit) is equal to 1 µg

It is an object of the present invention to provide new and improved oral formulations of lipidic substances showing better oxidative profile over known formulations. According to a first aspect of the present invention, there is provided an edible composition comprising castor oil and linseed oil. The composition of the invention comprising the combination of castor and linseed oils, preferably in defined ranges as disclosed below, is useful as lipid-based drug delivery (LBDD) system to provide/administer lipidic (lipophilic) substances, preferably lipidic drugs or lipidic dietary/food supplements otherwise difficult to deliver.

It is widely known that metabolic products of vegetable oils can cause an increase of oxidative stress, which in turn may induce serious tissue damage.

The castor oil and linseed oil composition of the invention is particularly advantageous in that in an in vitro system, cell viability upon cell treatment with said oils individually is higher than cell viability upon treatment with other oils commonly used for oral administration and formulation of lipidic substances, such as olive oil. Furthermore, cell viability was further improved when castor oil and linseed oil were mixed in different volumetric proportions. Notably, the production of ROS induced by the castor oil and linseed oil of the invention in the same experimental model is significantly less than the increase in ROS production caused by use of olive oil or pure castor oil and pure linseed oil.

Thus, in a particular embodiment of the present (emdodiment A)disclosure, there is provided an edible composition comprising castor oil and linseed oil characterized in that the castor oil: linseed oil volumetric ratio lies from 30:70 to 5:95.

In another particular embodiment, (embodiment B) the present invention provides an edible composition comprising castor oil and linseed oil characterized in that the castor oil: linseed oil volumetric ratio is about 25:75. In addition, the optimized mixture was chosen basing on the molecules added with the oils, but the range useful was from 40:60 to 10:90. It is noteworthy that the better efficacy of the mixed oils can be obtained using nanoemulsion-based optimized protocol as disclosed below.

In another embodiment (embodiment C), the above described embodiments A or B further comprise a first additional ingredient selected from the list of lipophilic vitamins, poliphenols, flavonoids, bioflavonoids, phytoestrogens, carotenoids, serenoa repens extracts, PDE5 inhibitors, Coenzyme Q10, and melatonin. According to a preferred embodiment of the invention, the compositions comprising the combination of castor and linseed oils, in ratio of 25:75, further comprise at least one additional ingredient selected from: lipophilic vitamins, preferably vitamin D, more preferably VD3, poliphenols, preferably resveratrol, flavonoids, bioflavonoids, phytoestrogens, carotenoids, serenoa repens extracts, PDE5 inhibitors, Coenzyme Q10, acetylsalicylic acid, melatonin and any combination thereof.

Suitably, the lipophilic vitamins are selected from the list of Vitamin A, Vitamin D, Vitamin E, Vitamin K. In a particular embodiment (Embodiment D), the lipophilic vitamin is Vitamin D3.

Poliphenols constitute a family of natural substances distributed widely in plant kingdom, produced as secondary metabolites by plants and so far 8000 representatives of this family have been identified. Increasing interest in polyphenols stems from the evidence of their role in prevention of degenerative diseases such as neurodegenerative diseases, cancer, and cardiovascular diseases. Natural polyphenols are a heterogeneous class of polyphenolic compounds contained in vegetables, which are being investigated as pharmaceutical compositions for the treatment of different metabolic disorders. Although the beneficial effect of these compounds has traditionally related to their antioxidant properties, they also exert several beneficial effects on hepatic and extra-hepatic glucose and lipid homeostasis. Furthermore, natural polyphenols exert antifibrogenic and antitumoural effects in animal models, which appear relevant from a clinical point of view. Several polyphenols, such anthocyanins, curcumin and resveratrol and those present in coffee, tea, soy are available in the diet and their consumption can be proposed as part of a healthy diet. Those and others such as as silymarin, are commonly consumed worldwide as nutraceuticals or food supplements for their antioxidant properties. Biomedical applications of these natural compounds are however severely hindered by their low bioavailability, rapid metabolism, and often by unfavourable physico-chemical properties, e.g. a generally low water solubility.

In one embodiment, polyphenols are selected from the list of flavonoids such as bioflavonoids, isoflavonoids, neoflavonoids. Of particular interest for the present invention are polyphenol compounds selected from anthocyanins, curcumin, resveratrol, sylmarin, and quercetin.

In one embodiment, the bioflavonoid is quercetin, a natural protective bioflavonoid, which possesses diverse pharmacologic effects, such as antioxidant, anti-inflammatory, anti-proliferative, and anti-angiogenic activities. Recently, quercetin effect in cancer prevention and treatment was recognized. However, the poor water solubility and low-bioavailability of quercetin limit its clinical use in cancer therapy.

In another embodiment, the bioflavonoid is resveratrol, or 3,5,4'-trihydroxy-trans-stilbene, produced naturally by several plants in response to injury or when the plant is under attack by pathogens such as bacteria or fungi. It has been found to potentially exhibit anticancer, antiangiogenic, immunomodulatory and cardioprotective activities as well as being an antioxidant. This is in addition to its usefulness in the treatment of neurodegenerative disease, diabetes and cardiac ailments. Currently, various studies have revealed that resveratrol is a potential drug candidate with multi-spectrum therapeutic application. However, the generally low solubility, stability, bioavailability and target specificity, together with the side effects seen when used at high levels, have limited their application.

Curcumin is a principal polyphenolic curcuminoid extracted from turmeric rhizome, which has been used for treating inflammation of joints, ulcers, jaundice and other disorders in Asian traditional medicine. It was found to have anti-inflammatory, antioxidant, anticarcinogenic, antimutagenic, anticoagulant, antifertility, antidiabetic, antibacterial, antifungal, antiprotozoal, antiviral, antifibrotic, antivenom, antiulcer, hypotensive and hypocholesteremic activities. The main problem associated with the use of curcumin as a chemopreventive agent in humans is its low absorption from the gastrointestinal tract, poor solubility in body fluids and low bioavailability. Phytoestrogens are a diverse group of naturally occurring nonsteroidal plant compounds that, because of their structural similarity with estradiol (17-β-estradiol), have the ability to cause estrogenic and/or antiestrogenic effects. Evidence is accruing that phytoestrogens may have a protective function against diverse health disorders, suchas prostate, breast, bowel, and other cancers, cardiovascular disease, brain function disorders and osteoporosis.

In one embodiment, the phytoestrogen is an isoflavone. Isoflavones are characterized by potent estrogenic activity; genistein, daidzein and glycitein are the most active isoflavones found in soybeans. The chemical form in which isoflavones occur is important because it influences their bioavailability and, therefore, their biological activity. Glucose-conjugated isoflavones are highly polar, water-soluble compounds. They are hardly absorbed by the intestinal epithelium and have weaker biological activities than the corresponding aglycone. Different microbial families of colon can transform glycosylated isoflavones into aglycones. Clinical studies show important differences between the aglycone and conjugated forms of isoflavones such as genistein and daidzein. Lipid-based formulations such as drug incorporation into oils, emulsions and self-microemulsifying formulations have been introduced to increase bioavailability. In a particular embodiment, the phytoestrogens are selected from the list of genistein and daidzein.

Oxidative stress is an important contributor to the risk of chronic diseases. Dietary guidelines recommend increased consumption of fruits and vegetables to combat the incidence of human diseases such as cancer, cardiovascular disease, osteoporosis and diabetes. Fruits and vegetables are good sources of antioxidant phytochemicals that mitigate the damaging effect of oxidative stress. Carotenoids are a group of phytochemicals that are responsible for different colors of the foods. They are recognized as playing an important role in the prevention of human diseases and maintaining good health. In addition to being potent antioxidants some carotenoids also contribute to dietary vitamin A. There is scientific evidence supporting the beneficial role of phytochemicals in the prevention of several chronic diseases. Although the chemistry of carotenoids has been studied extensively, their bioavailability, metabolism and biological functions are only now beginning to be investigated. Astaxanthin, beta-carotene, crocetin, lycopene, lutein, zeaxanthin are the most important carotenoids that have beneficial effects on human health. For example, lutein and zeaxanthin may be protective in eye disease. Most of the carotenoid pigments present in nature are quite water-insoluble. As such, one would expect to find them associated with the hydrophobic environment in biological systems. Carotenoids are used as cancer prevention agents, ulcer inhibitors, life extenders, and heart attack inhibitors. In a particular embodiment, the carotenoids are selected from the list of lutein, astaxanthin and zeaxanthin. Lipid-based formulations such emulsions formulations have been introduced to increase bioavailability at intestinal level.

The extract of the berry of the American saw palmetto, or dwarf palm plant, Serenoa repens (SeR), which is also known by its botanical name of Sabal serrulatum. The fruits of the saw palmetto are enriched highly with fatty acids and phytosterols, extracts of the fruits have been the subject of intensive research for the symptomatic treatment of benign prostatic hyperplasia. Serenoa Repens extracts reduce inflammation and decreases in vivo the androgenic support to prostatic cell growth. Furthermore, SeR extracts stimulate the apoptotic machinery.

Selenium (Se), an essential trace element mainly functioning through selenoproteins and able to promote an optimal antioxidant/oxidant balance, and lycopene (Ly), a dietary carotenoid synthesized by plants, fruits, and microorganisms with a strong antioxidant activity, has been shown to exert beneficial effects in prostate disease. SeR is frequently associated with lycopene, lycopene (Ly), a dietary carotenoid synthesized by plants, fruits, and microorganisms with a strong antioxidant activity and Selenium, an essential trace element mainly functioning through selenoproteins and able to promote an optimal antioxidant/oxidant balance, in attempt to increase its therapeutic activity in benign prostatic hyperplasia (BPH). It has been shown that the combination with Lycopene, Selenium, SeR has a greater and enhanced antiinflammatory activity that might be of particular interest in the treatment of BPH.Due to the lipophilic nature of the components, suitable formulations would include those based on vegetable oils.

PDE5 inhibitors promote vasodilative effects by enhancing intracellular cGMP levels. Molecules belonging to this class of compounds have been approved and commercialized as pharmaceutical compositions for therapy of various diseases such as pulmonary arterial hypertension (PAH), Raynaud's disease, gastrointestinal disorders stroke, erectile dysfunction, and furthermore exert cardioprotective effects. In one embodiment, the PDE5 inhibitors are selected from the list of Tadalafil, sildenafil, and vardenafil.

Coenzyme Q10 (CoQ10), also known as ubiquinone or ubidecarenone, is a powerful, endogenously produced, intracellular lipophilic antioxidant. It combats reactive oxygen species (ROS) known to be responsible for a variety of human pathological conditions. In case of deficiency and/or aging, CoQ10 is provided via oral supplementations a food supplement or dietary supplement. However, CoQ10 has low oral bioavailability due to its lipophilic nature, large molecular weight, regional differences in its gastrointestinal permeability and involvement of multitransporters. Therefore, the possibility to use a mixture of oils with high biocompatible properties for the cells could make the product greatly soluble and bioavailable.

Melatonin, chemically known as N-acetyl-5-methoxytryptamine, is a substance found in animals, plants, fungi and bacteria. In animals, it is a hormone that anticipates the daily onset of darkness; In animals, melatonin is involved in the synchronization of the circadian rhythms of physiological functions including sleep timing, blood pressure regulation, seasonal reproduction among others. Many of melatonin's biological effects in animals are produced through activation of melatonin receptors, while others are due to its role as a pervasive and powerful antioxidant, with a particular role in the protection of nuclear and mitochondrial DNA. Melatonin acts to stabilize cell membranes, thereby making them more resistant to oxidative attack. Melatonin is devoid of pro-oxidant actions. In experimental models of oxidative stress, melatonin has been shown to resist lipid peroxidation induced by paraquat, lipopolysaccharide, ischemia-reperfusion, L-cysteine, potassium cyanide, cadmium chloride, glutathione depletion, alloxan, and alcohol ingestion. Likewise, free radical damage to DNA induced by ionizing radiation, the chemical carcinogen safrole, lipopolysaccharide, and kainic acid are inhibited by melatonin. These findings illustrate that melatonin, due to its high lipid solubility and modest aqueous solubility is able to protect macromolecules in all parts of the cell from oxidative damage. Hence, a vegetable oil based formulation of melatonin would be highly beneficial.

Advantageously, the edible composition of the invention comprising castor oil, linseed oil, and Vitamin D3, has been shown by the present inventors to have an improved profile on cellular functions, with a better influence on cellular integrity, and an optimized anti oxidative effect on tissues expressing the VDR, when compared to commercially available VD3 as well as other formulations of VD3. Of particular interest is that Vitamin D3 of the present invention has an improved bioactivity profile when compared to commercially available Vitamin D3 formulations as measured by increased cell viability, cell proliferation and VDR levels in an in vitro experimental system. Furthermore, Vitamin D3 of the present invention demonstrates improved beneficial effects in that ROS production, in an in vitro experimental system, is lower when compared to other commercially available Vitamin D3 products.

According to a preferred embodiment of the invention, the composition is in form of nanoemulsion preferably prepared according to the method disclosed below.

In a second aspect of this invention, there is provided the compositions of embodiment D for use in treating a disease for which a therapeutic role of Vitamin D has been recognized. Non limiting examples of conditions which can be usefully treated with the Vitamin D3 composition of the invention are cardiac disease, stomach ulcer, liver damage, stroke, kidney insufficiency, inflammatory disease and malabsorption syndrome. Furthermore, Vitamin D3 compositions of the invention can be useful aids as to improve the individual antibody reaction, to reduce inflammation and as antioxidant.

In a third aspect to this invention there is provided the compositions of embodiment C or D for use in the manufacture of nutraceuticals, dietary supplements, food supplements, or medicaments. In other words, the compositions of the invention are for use as a medicament and/or as nutraceuticals and/or food supplements.

In a fourth aspect of this invention, there is provided the compositions of the invention, preferably embodiment D, further comprising a second additional ingredient selected from the list of acetylsalicylic acid, lipophilic vitamins, carotenoids, polyphenols, flavonoids, bioflavonoids, Coenzyme Q10, melatonin and metformin. Metformin, a biguanide derivative, is the most commonly prescribed medication in the treatment of type 2 diabetes mellitus. Recently, the use of metformin has shown potential as a preventive and therapeutic agent for a broad spectrum of conditions, including liver disease and hepatic malignancies. Metformin has also been observed to exhibit anti-thrombotic properties in insulin-resistant animal models. The pharmacologic effects of metformin are primarily exerted in the liver, at least partly via the activation of AMPK and the subsequent inhibition of gluconeogenesis. It has become increasingly clear that membrane transporters are important determinants of the pharmacokinetics of metformin. The compositions of embodiments A or B could act as transporters capable of conveying metformin through the membranes improving its pharmacokinetics.

In a particular embodiment (embodiment E), the second ingredient is acetylsalicylic acid.

In more specific embodiments of any aspect to this invention, the amount of Vitamin D3 lies from 5µg/ml to 125 µg/ml.

In another specific embodiment of any aspect to this invention, the amount of Vitamin D3 is equal to the recommended daily dose intake of Vitamin D3, raging from 400IU to 5000IU.

In a particular embodiment, the amount of acetylsalycilic acid (ASA) in the compositions of the present invention lies from 0.05µmol/ml to 20µmol/ml, eg from 10 µg to 650 µg.

The efficacy of several examples of the compositions of the invention has been confirmed by the applicant by using animal models (see below the examples). In particular, the inventors infused direct on blood the compositions and monitored the effects to exclude cytotoxicity. The concentration administered was calculated basing on FDA conversion formula from human to animal, based upon metabolic constant (Km).

Advantageously, the edible composition of the invention comprising castor oil, linseed oil, resveratrol, Q10, melatonin and/or Vitamin D3 and ASA has been shown by the present inventors to have an improved safety profile when compared to the same treatment without oils.

ASA is a well known anti inflammatory drug which exerts its anti-inflammatory effects through multiple mechanisms of action that include generation of reactive oxygen species (ROS), and increase of oxidative stress.

Many pathologies of stomach and liver are caused by oxydative injury, via ROS or Nitric oxide, and adverse effects observed in liver and stomach by long term pharmacological treatment with the same drugs (such as ASA) have been linked to increase of the oxidative stress condition

Among the non-classical mechanisms of action for Vitamin D3, such as apoptosis inhibition and improvement of autophagy and survival mechanisms, prevention of oxidative damage in various tissues has been demonstrated.

In particular, the present inventors have surprisingly found that VD3 when dissolved in a 75%linseed oil/25% castor oil mixture improves cell survival upon oxidative stress, and has a protective role on cells during oxidative stress obtained with H2O2 treated with ASA, by decreasing ROS production.

Compositions of the present invention can be formulated as single dose form. Suitably, the formulation may be packaged in a powder, tablet, gelatin pearl, or a vial. A further aspect of this invention, there is provided compositions of Embodiment E for use for the treatment of oxidative stress to prevent the risk of chronic diseases, and to improve the health during aging. The compositions of Embodiment E are useful for gynecological disorders, inflammatory diseases such as osteoarthritis, rheumatoid arthritis, myocardial infarction, ischemic stroke, neuronal degeneration, anti-cancer support. The compositions of Embodiment E are also useful for dietary support, the prophylaxis of thromboembolic stroke or revascularization procedures. The compositions of Embodiment E are particularly useful for subjects suffering of a disease selected from the following list: kidney failure, gastric ulcer, hemostasis impairment, cardiovascular stroke, brain stroke, liver insufficiency, iatrogenic diseases, eye diseases, skin diseases, autoimmune diseases or subjects undergoing anti clotting therapy, anti thrombosis therapy, suffering from adverse effects of chemotherapy or needing support to chemotherapy, recovery after heart surgery, or a post trauma therapy.

Resveratrol, Q10, Melatonin in the embodiments are used in the human therapeutic range (50µM), particularly in animal study Q10 concentration was converted following the FDA conversion from human to animal basing on metabolic constant (Km).

A further aspect of the present invention refers to a process for preparing a nanoemulsion comprising the compositions according to the present invention.

The process involves at least one, preferably 1-10, more preferably 2-5 steps of homogenizing the combination of castor and linseed oils. Preferably, the amount of castor oil ranges from 15% to 25% and/or the amount of linseed oil ranges from 85% to 75% being said percentage grams per 100mL. The oils are used in the ranges disclosed before, and, preferably, the oil with higher percentage is added to the homogenizer first. The homogenizing step allows the dispersion of the oils and is performed at low temperature. In this context, low temperature means under cooling condition, such as around 4°C for example on the ice. In any case, it is advisable to monitor the temperature of the process and to keep it under 37°C.

Preferably, a homogenizer with rod for dispersion (VELP) is used.

According to a preferred embodiment of the invention, the homogenizing step is performed at 1000-5000 rpm, preferably 1500-3000 rpm, more preferably about 2000 rpm.

The process comprises preferably a further step wherein at least one additional ingredient is added to the combination of oils before homogenizing wherein said ingredient is selected from: lipophilic vitamins, poliphenols, flavonoids, bioflavonoids, phytoestrogens, carotenoids, serenoa repens extracts, PDE5 inhibitors, Coenzyme Q10, melatonin and any combination thereof. Preferably the additional ingredient is selected from resveratrol and/or Coenzyme Q10 and/or melatonin and/or lipophilic vitamins preferably vitamin D, more preferably VD3.

The process allows to obtain a stable nanoemulsion that, according to a preferred embodiment, can be added in drops to a gelatin solution to solidify. Preferably, the gelatin solution comprises 10-30% gelatin, more preferably about 20% gelatin. The gelatin solution is preferably pre-warmed to allow the gelatin becomes melted. Alternatively, it is possible to solidify the composition of the invention by using maltodextrin as alternative to the gelatin. Moreover, dry congealing or silica gel are alternative methods.

Therefore, a further aspect of the present invention refers to the nanoemulsion obtained/obtainable by using the method disclosed above. This nanoemulsion can be used for medical purposes or as nutraceutical product or dietary supplements in the same way as disclosed before for the composition.

### Examples of the invention

*Cell lines and culturing methods:* HuH-7 is a well differentiated hepatocyte derived cellular carcinoma cell line that was originally taken from a liver tumor in a 57-year-old Japanese male in 1982. HuH-7 is an immortal cell line of epithelial-like tumorigenic cells. Huh-7 is widely used as an in vitro system to study hepatotoxicity. It usually grows in 2D monolayers in Dulbecco's modified Eagle medium (DMEM) containing 10% fetal bovine serum (FBS), 2mM L-glutamine, and 1% penicillin-streptomycin at 37°C in an incubator at 5% CO₂. Before the experiments cells were washed and incubated for 2-4h in DMEM without red phenol and supplemented with 0.5% FBS, 2mM L-glutamine, and 1% penicillin-streptomycin at 37°C in incubator and then stimulated. The cells were used to performed MTT and Crystal violet tests plating 1x104 cells in a 96 well-plates; to study ROS production plating 1x10⁴ in a 24-well plates; to analyzed Annexin V plating 0.2 x10⁴ cells in a chamber slide; to make a Western blot analysis plating in a 35mm dish until the confluence.

GTL-16 cells are a highly tumorigenic cell line clonally derived from MKN-45 cells. The latter were originally established from a poorly differentiated gastric adenocarcinoma. The GTL-16 line is a gastric tumor cell line commonly used as a model to examine tolerability of gastric apparatus in human and is cultured in Dulbecco's Modified Eagle Medium (DMEM, Sigma-Aldrich, Milan Italy) medium supplemented with 10% Foetal Bovine Serum (FBS, Sigma-Aldrich, Milan Italy), 2mM L-glutamine (Sigma-Aldrich, Milan Italy), and 1% penicillin-streptomycin (Sigma-Aldrich, Milan Italy) at 37°C in an incubator at 5% CO₂. The cells were used to perform different experiments, such as peroxidation study and quantification of vitamin D, plating 1x10⁶ cells in 6 well-plates, MTT test plating 1x10⁴ cells in a 96 well-plates; to study ROS production plating 1x10⁴ in a 24-well plates.

Before the experiments cells were washed and incubated for 2-4h in DMEM without red phenol (Sigma-Aldrich, Milan Italy) and supplemented with 0.5% FBS, 2mM L-glutamine, and 1% penicillin-streptomycin at 37°C in incubator and then stimulated. The cells were used to make MTT test plating 1x10⁴ cells in a 96 well-plates; to study ROS production plating 1x10⁴ in a 24-well plates.

Caco-2 cells are a human colon epithelial cancer cell line used as a model of human intestinal absorption of drugs and other compounds and are applied to predict the intestinal absorption following oral exposure. This is the most used cell model to study absorption, metabolism, and bioavailability of drugs and xenobiotics. Notwithstanding it, represents a well-established and accepted in vitro model for the human intestinal epithelium and its use is recognized by the. The Caco-2 cell population maintained in culture is characterized by an intraclonal variability revealed by the presence of subpopulations with different morphologies and different physiological characteristics. This property of Caco-2 cells led to the existence of different sub-lines, often of clonal origin maintained in different laboratories. However, it is widely reported in literature that culture-related conditions, as well as the different Caco-2 cell lines utilized in different laboratories, often used a new cell-maintenance protocol in which Caco-2 cells were subcultured at 50% of confluence instead of 80% of confluence, as usually suggested. These cells were grown in Dulbecco's Modified Eagle's Medium/Nutrient F-12 Ham (DMEM-F12, Sigma-Aldrich, Milan Italy) containing 10% fetal bovine serum (FBS, Sigma-Aldrich, Milan Italy), 2mM L-glutamine (Sigma-Aldrich, Milan Italy), and 1% penicillin-streptomycin (Sigma-Aldrich, Milan Italy) at 37°C in an incubator at 5% CO₂. The cells were used to perform different experiments, such as MTT, plating 1x10⁴ cells in a 96 well-plates, ROS production analysis plating 1x10⁴ in a 24-well plates, peroxidation study and Western blot plating 1x10⁶ cells in 6-well plates, quantification of vitamin D, plating 1x10⁶ cells in 6-well plates, absorption study plating 20000 cells on 6.5 mm Transwell with 0.4 µm pore polycarbonate membrane insert (Sigma-Aldrich, Milan Italy) in a 24-well. Before the experiments, except for transwell protocol, cells were washed and incubated for 8h in DMEM without red phenol and supplemented with 0.5% FBS, 2mM L-glutamine, and 1% penicillin-streptomycin at 37°C in incubator and then stimulated. The cells plated on transwell insert, were maintained in complete medium changed on alternate days, first basolaterally and then apically for 21 days before the stimulations.

CHO cells (Chinese hamster ovary) are an epithelial cell line derived from the ovary of the Chinese hamster, often used in biological and medical research and commercially in the production of therapeutic proteins. They have found wide use in studies of genetics, toxicity screening, nutrition and gene expression, particularly to express recombinant proteins. CHO cells are the most commonly used mammalian hosts for industrial production of recombinant protein therapeutics. The CHO cell line was derived as a subclone from the parental CHO cell line initiated from a biopsy of an ovary of an adult Chinese hamster by T. T. Puck in 1957. The cells are cultured in Dulbecco's Modified Eagle Medium (DMEM, Sigma-Aldrich, Milan Italy) medium supplemented with 10% Foetal Bovine Serum (FBS, Sigma-Aldrich, Milan Italy), 2mM L-glutamine (Sigma-Aldrich, Milan Italy), and 1% penicillin-streptomycin (Sigma-Aldrich, Milan Italy) at 37°C in an incubator at 5% CO₂. The cells were used to make MTT test plating 1x10⁴ cells in a 96 well-plates; to study ROS production plating 1x10⁴ in a 24-well plates, to Western blot and quantification study the cells were plated in a 6-well till to the 90% of confluence. Before the experiments cells were washed and incubated for 2-4h in DMEM without red phenol (Sigma-Aldrich, Milan Italy) and supplemented with 0.5% FBS, 2mM L-glutamine, and 1% penicillin-streptomycin at 37°C in incubator and then stimulated.

*H9c2 cell culture:* Recent studies demonstrated that cultured primary cardiomyocytes are a valuable tool for studying the metabolic capacity of the heart. However, a major limitation for isolated cardiomyocytes is that they are rather fragile and difficult to isolate. Therefore, is important provided useful alternative, using in vitro cell line model H9c2. The H9c2 cell line was originally derived from embryonic rat ventricular tissue and they still show many similarities to primary cardiomyocytes, including membrane morphology, signalling protein expression and electrophysiological properties. This cell line offers a valuable in vitro model to study the metabolic capacity of the heart and to investigate the pathogenesis of various cardiovascular diseases. This cell line is cultured in Dulbecco's Modified Eagle Medium (DMEM, Sigma-Aldrich, Milan Italy) medium supplemented with 10% Foetal Bovine Serum (FBS, Sigma-Aldrich, Milan Italy), 2mM L-glutamine (Sigma-Aldrich, Milan Italy), and 1% penicillin-streptomycin (Sigma-Aldrich, Milan Italy) at 37°C in an incubator at 5% CO₂.The cells were used to make MTT test plating 1x10⁴ cells in a 96 well-plates; to study ROS production plating 1x10⁴ in a 24-well plates; to Western blot analysis the cells were plated in a 6-well plates and maintained in a complete medium till to the 90% of confluence.

Before the experiments cells were washed and incubated for 2-4h in DMEM without red phenol (Sigma-Aldrich, Milan Italy) and supplemented with 0.5% FBS, 2mM L-glutamine, and 1% penicillin-streptomycin at 37°C in incubator and then stimulated.

*Experimental protocol:* HuH-7 and GTL-16 were stimulated with the same agents for 24h at 37°C in a incubator. Vitamin D3 (Sigma-Aldrich, Milan Italy) was dissolved in different solvent such as ethanol (Sigma-Aldrich, Milan Italy); castor oil (CARLO ERBA OTC SpA, Italy), linseed oil (ACEF SPA, Italy) and olive oil (virgin olive oil, mechanically extracted) to analyze the hypothetic synergistic effects of Vitamin D and solvent. In these experiments the pure oil-based solvent was tested alone (100%) or in combination with vitamin D3 and the results were compared to the commercial vitamin D3, which is dissolved refined olive oil, (named V dibase, Abiogen Pharma, Italy). Since the efficacy of pure linseed and castor oils combined with vitamin D3 was significantly compared to control, in the second set of experiments different dilutions of linseed and castor oil alone were tested to determine the best concentration of oil in the solvent. This is important to define the range to use the mixture oils with different agents. In the third part of experiments, a mixture of different oils was tested alone and in presence of Vitamin D3: 25% castor oil mixed with 75% linseed oil; 75% castor oil mixed with 25% linseed oil; 50% castor oil mixed with 50% linseed oil. These conditions were tested to determine the influences of oils on Vitamin D3 effects and the best combination able to induce the maximum effects was maintained for all successive experiments. In addition we have also tested the effects of commercial Vitamin D (V di base) compared to the new preparation.

In addition, GTI-16, Caco-2, H9c2 and CHO cells were used to test the efficacy of linseed and castor oils in different mixture in presence of resveratrol, Q10 and melatonin with or without vitamin D3. These experiments are important to study the properties of mixture oils to solubilize the lipophilic substances and to understand the better conditions to use the mixture with these substances. In addition, they can explain the importance of vitamin D3 to enhance the effectiveness of antioxidant substances.

### MTT test

In Vitro Toxicology Assay Kit (Sigma-Aldrich, Milan Italy), MTT based, was used to determine cell viability on Huh-7 and GTI-16 cell lines. After the stimulations the cells were incubated with DMEM without red phenol and FBS containing 1% MTT dye for 2 h at 37°C in incubator. Then, the medium was removed and the purple formazan crystals (3-[4,5-dimethylthiazol-2-y1]-2,5-diphenyltetrazoliumbromide) were dissolved in equal volume to the original culture medium of MTT Solubilization Solution. Cell viability was determined by measuring the absorbance through a spectrometer (VICTORX3 Multilabel Plate Reader) at 570nm with correction at 690nm, and cell viability calculated by comparing results to control cells (100% viable).

*ROS production:* The rate of superoxide anion release was used to examine the involvement of Vitamin D3 with oils and of oxidative stress in the mechanism activated by Vitamin D3 and ASA on both cell type in presence or absence of H2O2. The superoxide anion production was measured as superoxide dismutase-inhibitable reduction of cytochrome C. In all samples (stimulated and untreated), 100µL of cytochrome C (Sigma-Aldrich, Milan Italy) was added, and in another one, 100 µL of superoxide dismutase (Sigma-Aldrich, Milan Italy) was also added for 30 minutes in an incubator. The absorbance changes in the supernatants of the sample was measured at 550 nm in a Wallac Victor model 1421 spectrometer (PerkinElmer). The O2 was expressed as nanomoles per reduced cytochrome C per microgram of protein, using an extinction coefficient of 21 000 mL/cm, after the interference absorbance subtraction.

*Crystal violet staining method:* To study cell proliferation, the same experiments were replicated using crystal violet staining on Huh-7 cells. After each treatment the medium was removed and the cells were washed with PBS 1X (Sigma-Aldrich, Milan Italy), fixed with 1% glutaraldehyde (Sigma-Aldrich, Milan Italy) for 15 min at room temperature (RT), washed twice with PBS 1X, and stained with 100µL of 0.1% aqueous crystal violet (Sigma-Aldrich, Milan Italy) for 20 min at RT. The multi-well plates were rinsed four times in tap water and allowed to dry. To obtained cell number estimation were added to multi-well plates 100 µl of 10% acetic acid (Sigma-Aldrich, Milan Italy), and the content of each well was mixed before reading the absorbance at 595nm through a spectrometer (VICTORX3 Multilabel Plate Reader). The number estimation was calculated by comparing results to control cells. *Peroxidation analysis:* Lipid peroxidation is the degradation of lipids that occurs as a result of oxidative damage and is a useful marker for oxidative stress. Polyunsaturated lipids are susceptible to an oxidative attack, typically by reactive oxygen species, resulting in a well-defined chain reaction with the production of end products such as malondialdehyde (MDA). Lipid peroxidation may contribute to the pathology of many diseases including atherosclerosis, diabetes, and Alzheimer's. Lipid peroxidation is determined by the reaction of MDA with thiobarbituric acid (TBA) to form a colorimetric product, proportional to the MDA present, using Lipid Peroxidation (MDA) Assay Kit (Sigma-Aldrich). GTL-16 and Caco-2 cells, after treatments with mixture oils alone and in presence of vitamin D3, were homogenized on ice in 300 µL of the MDA Lysis Buffer containing 3 µL of BHT (100x) for each sample. After centrifugation at 13000 rpm for 10 minutes, 200 µL of the supernatant from each samples and 600 µL of the TBA solution were incubated at 95°C for 60 minutes. The sample were analyzed in a 96 well-plate and the absorbance were measured at 532 nm. For the quantification of lipid peroxidation in necessary, plot a standard curve.

*Quantification of vitamin D3 in cells:* Vitamin D₃ the active form of vitamin D, has a very short-lived and is rapidly metabolized to the deactivated forms 24,25(OH)₂D₃ and 1,24,25(OH)₃D₃. For this reason the inventors have used a competitive EIA assay kit that primarily detects the more metabolically stable forms, 25(OH)D₃ and 25(OH)D₂ (Cayman's Vitamin D EIA Kit). In GTL-16 and Caco-2 after the treatments with vitamin D3 prepared in different mixture of castor and linseed oils and with D-Base, the medium was removed and the cells mechanically lysed in ice PBS 1X (Sigma-Aldrich, Milan Italy), crashed through three cycles of freeze-thaw, centrifuged at 4°C for 30 min at 13000 rpm and the supernatants were used for quantification analysis. Before adding to wells, equilibrate the SABC working solution and TMB substrate for at least 30 min at room temperature and washes the strips of the plate 2 times before adding standard, sample and control. For the quantification is necessary plot a standard curve including control (zero well). Add 0.1 ml of each sample and standard into test sample wells, seal the plate with a cover and incubate at 37 °C for 90 min. After the plate content was removed, and added 0.1 ml of Biotin-detection antibody work solution into the standard and test sample at 37°C for 60 min. After the plate was washed 3 times with Wash buffer and 0.1 ml of SABC working solution into each well was added and the plate incubated at 37 °C for 30 min. After the plate was washed 3 times with Wash buffer and 90 µl of TMB substrate into each well was added and the plate incubated at 37°C in dark within 15-30 min. After this time 50 µl of Stop solution into each was added and the absorbance measured at 450 nm in a microplate reader immediately after adding the stop solution.

*Quantification of Resveratrol in cells:* Recently, clinical studies have demonstrated several health-beneficial effects of trans-resveratrol, a natural food compound found in grape, such as anti-oxidizing, anti-atherosclerotic effects, inhibition of platelet aggregation, cardiovascular protective effects and chemoprotective advantages against cancer proliferation. However, resveratrol is rapidly metabolized in vivo. It also has low water solubility, which reduces the dissolution-rate limited cell absorption, leading to reduced oral bioavailability. The development of efficient drug delivery systems is still one of the most promising approaches. For this reason the inventors test absorption rate in CHO cells of resveratrol (Sigma-Aldrich, Milan Italy) mixed in optimized mixture of castor and linseed oils in presence or absence of vitamin D3. After treatments the medium was collected to analyze the extracellular resveratrol and the cells mechanically lysed in PBS 1X (Sigma-Aldrich, Milan Italy) using cell scraper, mixed for 20 min at 4°C in ice and centrifuged for 20 minutes at 13000 rpm at 4°C and the supernatants were analyzed to determine the intracellular fraction of trans-resveratrol. The quantification of trans-resveratrol was performed using a Shimadzu HPLC system (Shimadzu, Kyoto, Japan), consisting of two LC-10AD Vp module pumps, an SLC-10A Vp system controller, an SIL-10AD Vp autosampler, and a DGU-14-A on-line degasser. All the chromatographic separations were performed on a Phenomenex Luna PFP(2) 5µm (150 x 4.6 mm i.d.) (Torrance, CA, USA) as a stationary phase protected by a Luna PFP(2) SecurityGuard (Phenomenex). The SPD-M10Avp photodiode array and fluorescence RF-10Axl detectors were used to detect RES (360 nm) and RES-Dns (λₑₓ=314 nm, λₑₘ=525 nm) analytes respectively. LC Solution 1.24 software was used to process the chromatograms. The quantity of resveratrol was obtained comparing to standard curve.

*Annexin V analysis:* The immunostaining procedure was performed on Huh7 plated on chamber slide, using Annexin V (13µg/ml, Sigma-Aldrich, Milan Italy). After the stimulations the cells were fixed using ice-buffer PAF (3.7% formaldehyde, Sigma-Aldrich, Milan Italy, and 1% sucrose, Sigma-Aldrich, Milan Italy, in PBS 1X) for 20 min, washed three times with ice-PBS 1X and then permeabilized with ice 0.5% Triton X-100 (Sigma-Aldrich, Milan Italy) in PBS 1X for 20min at 4°C. Endogenous peroxidase activity was quenched during 8 min of incubation with 3% H2O2 (Marco Viti Farmaceutici S.p.A., Italy) in PBS 1X, and then blocked with 3% BSA (Sigma-Aldrich, Milan Italy) in PBS 1X for 1h. Slides were incubated overnight at 4°C with specific primary antibody diluted in PBS 1X and incubated with biotinylated "universal" secondary antibody for 20 min. Slides were then washed in PBS 1X, incubated for 20 min with streptavidin solution (VECTASTAIN® R.T.U. ABC Reagent, DBA Italia srl Italy) and developed with 3-3'diaminobenzidine (DAB), rinsed, counterstained with Mayer's hematoxylin (Sigma-Aldrich, Milan Italy) and mounted with aqueous mounting medium. The number of positive cells was calculated as described elsewhere: briefly, 12 different areas (1mm2) randomly selected from each section were taken, and the number of signals was determined using ImagePro 3 software (NIH, Bethesda, US). The results were expressed as a means±SD (%). *Western blot analysis:* Huh-7, GTL, and Caco-2 cells after stimulation were washed with ice-PBS 1x supplemented with 2mM sodium orthovanadate (Sigma-Aldrich, Milan Italy), and lysed in ice Complete Tablet buffer (Sigma-Aldrich, Milan Italy) supplemented with 2mM sodium orthovanadate and 1:1000 phenylmethanesulfonylfluoride (PMSF, Sigma-Aldrich, Milan Italy), PIB inhibitors 1:100 and mix of protease 1:100. The proteins were quantified by using BCA (Thermo Fisher Scientific, Italy) and 35µg of proteins from each lysate were resolved on 15%, 8% or 4% SDS-PAGE gels and transferred to polyvinylidene fluoride membranes (PVDF, Sigma-Aldrich, Milan Italy) and then incubated overnight at 4°C with specific primary antibody anti-p53 (1:500, Santa-Cruz, Italy), anti p-ERK (1:1000, Euroclone SpA Italy), anti-Melatonin 1/2 receptor (1:250, Santa-Cruz, Italy), Santa-Cruz, Italy), anti-VDR receptor (1:250, Santa-Cruz, Italy) and Ki67 (1:250, Sigma-Aldrich, Milan Italy). The blottings were visualized by chemiluminescence using Western Lightning Plus ECL (Thermo Fisher Scientific, Italy) and the protein expression was normalized and verified through β-actin detection (1:5000, Sigma-Aldrich, Milan Italy).

*NO assay:* NO was measured as plasmatic nitrite level by the Griess system. Briefly, 5 ml of plasma was deproteinized by the addition of 10 ml of 35% sulfosalicylic acid. Treated samples were mixed by vortexing every 5 min and allowed to react for 30 min at room temperature. Then they were centrifuged at 10000 g for 15 min. Fifty microliters of the supernatant was added to 100 microliters of saline in a 1:2 dilution for subsequent analysis. The other microliters were used without any dilution. The samples were passed through a copper-cadmium column of an autoanalyzer (Autoanalyzer, Technicon Instruments Corp., Tarrytown, NY, USA) to reduce nitrate to nitrite. The plasma samples were mixed with equal volume of Griess reagents and after 10 min the absorbance at 570 nm was measured by spectrometer (BS1000 Spectra Count, San Jose, CA, USA).

*In vivo experiments:* The ability of mixture oils and its applicability was demonstrated in animal rats model using male Wistar rats weighing 350 to 400 g (n=42); the animals were housed in a room at a constant temperature of 25°C on a 12-hour/12-hour light/dark cycle with food and water available ad libitum. All experiments were conducted in accordance with local ethical standards and protocols approved by national guidelines (legislative decree (DLGS), January 27, 1992, license 116) and in accordance with Guide for the Care and Use of Laboratory Animals (National Institutes of Health publication 86-23, 1985 revision). For each animal, anesthesia was performed via sevoflurane (5% for induction and 3% for maintenance) in oxygen. A heat pad maintained the temperature of the animals. Under sterile conditions, rats underwent surgical procedure, thoracotomy, used to expose and isolate aorta to perform the injection and to measure blood flow, using flowmeter probe (model 420, Transonic Systems, Inc., Ithaca, NY) positioned around the vessel. Aortic blood was immediately collected at the end of treatments. The heart rate and respiratory rate were also continuously monitored. Aorta was a better choice to study cardiac function of drugs. Dose selection was based upon in vitro experiments and upon the study of the translation from animal to human. Thus, Q10 administration resulted in 12.5mg for in vitro experiments and 50mg in in vivo experiments. The time of treatments was chosen at 120 sec, respecting the classical acute protocol of administration. The animals were monitored all the time to keep the animal welfare and avoid adverse reactions.

*Solution preparations:* The mixture of castor and linseed oils was optimized depending on the molecules used. The range of castor oil resulted optimal in all protocol was summering from 15% to 25%of castor oil mixed with from 85% to 75% linseed oil, respectively. The emulsion was obtained using a homogenizer with rod for dispersion (VELP) at 2000 rpm maintaining cool-condition. The substances were added during cool-dispersion. The inventors want to create an alternative formulation, adding 20% gelatin bovine to solidify the oil solution. Alternatively, they hypothesized to introduced other techniques, such as maltodextrin, spray or dry congealing or using silica gel.

*Statistical analysis:* Results are expressed as means±SD of at least 5 independent experiments for each experimental protocol. Statistical comparisons between groups were made using One-way ANOVA with Bonferroni post hoc test or Mann-Whitney U test, as appropriate, using GraphPad Prism 5 (Graph Pad Software, La Jolla, CA, USA). P-value <0.05 was considered statistically significant.

Various formulations of lipophilic substances were tested in Huh-7, GTL-16, Caco-2, CHO and H9c2 cell lines. These experimental models are particularly relevant in order to clarify the effectiveness of a mixture of castor and linseed oils and to test the efficacy of Vitamin D3 (VD3) and to enhance the effectiveness of other lipophilic substances as well as vegetable oils, the metabolism of which involves stomach, intestine and liver in humans after oral intake. In addition, to demonstrate the beneficial effects of a mixture of vegetable oils, antioxidants and/or vitamins "in the whole organism" the inventors used different cell lines, such as ovarian and cardiac cells.

### Example 1

*Effects of different vegetable oils on cell viability and ROS production:* The effect of different vegetable oils on cell viability and Reactive Oxygen Species (ROS) production was analyzed in Huh-7 and GTL-16 cell lines. The results were expressed as means ±SD (%), normalized against control values.

The effect of the vegetable oils tested on cell viability in these cell lines is indicative of a possible beneficial effect on specific organs, namely stomach and liver.

Fig.s1A and 1B show the effect of linseed oil (O1), castor oil (O2) and olive oil (O3) on cell viability (MTT test), compared to Ethanol, in HuH-7 and GTL-16 cells, respectively. Pure linseed oil (O1) and castor oil (O2) show a similar positive effect on cell viability, better than olive oil (O3). Et-OH= ethanol; O1 100%= linseed oil; O2 100% = castor oil; O3 100%= olive oil 100%;

In Fig.1C, the effect on cell viability of compositions of different dilutions of linseed oil and castor oil were tested in HuH-7 cells, in order to determine a range of concentration of each oil for optimal effect on cell viability. Each oil was diluted directly in cell culture medium in order to obtain a final concentration ranging from 5% to 100% in oil.

The range of efficacy of linseed oil varied from 70% to 85%, whereas the range for castor oil concentration varied from 15% to 35%.

The two vegetable oils were mixed in different proportions, as shown in Fig.s1D and 1E, and tested on cell viability by the MTT test in both Huh-7 and GTL-16 cell lines, respectively. The best combination of the two oils was determined to be 75% linseed oil and 25% castor oil.

Metabolic products of vegetable oils can cause an increase of oxidative stress, which in turn may induce serious tissue damage, especially in organs such as stomach and liver. Therefore ROS production induced by linseed oil, castor oil and a mixture of the two oils compared to Ethanol (EtOH) and olive oil (O3) was examined in Huh-7 and GTL-16 cell lines by a colorimetric assay based on reduced cytochrome c (see methods).

In order to enhance the beneficial effect on cell viability of the mixture of oils in the concentration observed as the optimal ranging, the inventors have performed some experiments on GTL-16 using a nanoemulsioned mixture of oils, prepared as reported in methods. The effect of the vegetable oils tested on cell viability in these cell lines is indicative of a possible beneficial effect on stomach after oral intake. Each oil was diluted directly in cell culture medium in order to obtain a final concentration ranging from 5% to 100% in oil. The range of efficacy of linseed oil varied from 65% to 90%, whereas the range for castor oil concentration varied from 10% to 35%, as shown in Fig.2A. The two vegetable oils were mixed in different proportions, as shown in Fig.2B and the best combination of the two oils was confirmed to be 75% linseed oil and 25% castor oil. In addition, the beneficial effect obtained by nanoemulsion was demonstrated starting from 60% of linseed oil with 40% castor oil to 90% of linseed oil with 10% castor oil compared to the same mixed prepared without nanoemulsion. These data are important because the inventors can apply an optimal mixture oils based upon the substances added.

As reported in Fig.s3A (Huh-7) and 3C (GTL-16), cell treatment with linseed and castor oil showed increased ROS production in both cell lines, as expected. However, the increase in ROS production was significantly less than the increase caused by treatment with olive oil (O3), widely used as solvent for hydrophobic substances for cosmetic, nutraceutical and pharmacological use. Next, a mixture made of 75% linseed oil and 25 % castor oil was tested for ROS production in HuH-7 and GTL-16 cells. As shown in Fig.s3B (Huh-7) and 3D (GTL-16), a significant reduction in ROS production was observed in presence of the mixture of the two vegetable oils, compared to pure oils in both cell lines. Finally, in GTL-16, the beneficial effects exerted by mixture of oils on ROS production were also observed with the mixture of oils prepared as a nanoemulsion (Fig.2C). A significant beneficial effect (p<0.05) can be observed starting from 70% linseed oils with 30% castor oils to 90% linseed oils with 10% castor oils compared to the same concentration prepared without nanoemulsion. For this reason the nanoemulsion protocol was maintained for successive experiments in presence of additional substances.

*Effects of different oils on ERK*/*MAPK expression and cell proliferation:* The effects of the vegetable oils, either pure or mixed with each other, on intracellular pathways related to cell proliferation and prevention of ROS formation were examined in HuH-7 cells.

Cell proliferation is considered to be an important parameter to determine the integrity of a tissue, since when a tissue damage occurs, the tissue (such as stomach and liver) spontaneously repairs itself by proliferation and migration of the cells. Hence, the effect of the vegetable oils tested on cell proliferation is considered a parameter of the influence of said oils on tissue integrity.

The ability to proliferate of Huh-7 cells treated with vegetable oils was investigated by crystal violet assay, which allows counting the number of cells after the treatment, as well as by Western blot and densitometric analysis of KI67, which is a well known cellular protein marker of proliferating cells. Further, ERK^{1/2}/MAPK proteins, which are involved in regulation of cell migration and of cell survival, were measured by Western blot and densitometric analysis.

As shown in Fig.4A, the vegetable oils or the mix thereof did not influence cell number after 24h stimulation, compared to control. Similar data were observed upon KI67 staining for the mixed oils, as shown in Fig. 4B. These data demonstrate the absence of any alteration on cellular proliferation in absence of damage, indicating a balance in physiological functions.

As shown in Fig.s5A and 5B, expression of ERK^{1/2} was observed in presence of pure castor oil, indicating an alteration on cell cycle that may lead to apoptosis and eventually cell death, suggesting that use of pure castor oil as solvent ought to be avoided as possibly leading to tissue damage. The linseed oil alone or the 75% linseed oil/25% castor oil mixture showed minimal increase of expression of ERK^{1/2}, indicating a physiological activation induced by the oil without any alteration of physiological metabolism.

In order to exclude cell cycle alterations and cell death induction, p53 activation in presence of the vegetable oils and the 75% linseed oil/25% castor oil mixture was measured in Huh-7 cells by Western blot and densitometric analysis.

In unstimulated cells, p53 is expressed at very low levels; however, in response to DNA damage or other stress stimuli, p53 becomes stabilized and accumulates in the cells. Nuclear and cytoplasmic p53 also physically interact with many other proteins to promote apoptosis and other processes. For example mitochondrial p53 also regulates oxidative stress-induced necrosis through its interaction with CypD.

As shown in Fig.s5A and 5B, 100% pure castor oil caused a significant increase in p53 activation compared to control and to linseed oil alone, confirming that castor oil alone has a negative effect on cellular homeostasis. On the contrary, the 75% linseed oil/25% castor oil mixture induced a significant reduction of p53 activation compared to control and to pure oils.

*Absorption rate of different oils on intestinal cells:* Other important parameters that must be necessary considered if the mixture oils would be used in human, are the absorption rate and the peroxidative level of intestinal barrier. The small intestine is the primary organ responsible for the absorption and serves as a physical and biological barrier. Caco-2 cells originally isolated from human colon carcinoma, undergo spontaneous enterocyte differentiation in culture to resemble epithelial cells of the small intestine. For this reason, the inventors decided to use these cells as a model of human intestinal absorption, to predict the intestinal drug absorption in human and to study the drug transport. The beneficial effects exerted by nanoemulsion mixture oils (starting from 70% to 85%) were confirmed by the experiments on cell viability, absorption and peroxidation rate in Caco-2 cells. As reported in Fig.6A, the effect on cell viability of the different nanoemulsion mixture oils was time dependent with a maximum effect at 720 min and this time of stimulation was maintained in successive test on Caco-2. As reported in Fig.6B, a significant beneficial effects about the antioxidant property of the emulsion mixture oils can be observed starting from 75% linseed oils with 25% castor oils to 85% linseed oils with 15% castor oils (p<0.05) compared to control (untreated samples) on MDA concentration, the end product of of lipid peroxidation. Usually ROS can react with the polyunsaturated fatty acids of lipid membranes and induce lipid peroxidation, forming MDA. Antioxidative mechanisms (ROS release) and SOD activity were inversely proportional to MDA concentration. MDA has been widely used for many years as a convenient biomarker for lipid peroxidation of omega-3 and omega-6 fatty acids because of its facile reaction with thiobarbituric acid (TBA). The moderately high MDA levels (5 and 10 µM) promoted a potential DNA damage. As shown in Fig.6B, the rate of MDA was more less than µM concentration, supporting the beneficial antioxidant property of nanoemulsion mixture oils. Finally, to demonstrate the ability of nanoemulsion mixture oils to cross the intestinal membrane, a measurement of the down volumes of each concentrations at 720 min was performed. As reported in Fig. 6C, each nanoemulsioned mixture of oils was able to cross the membrane (volume - *µ*l) but the effectiveness was better starting from 75% to 85% than 70% (p<0.05) and control (p<0.05). The physiological condition and the integrity of the membrane was confirmed by augmented volume of control compared to T0 measurement (p<0.05). These data support the ability of the mixture to cross membrane and to use these mixture oils as a delivered for additional substances after oral intake.

For this reason, the nanoemulsion protocol was maintained for successive experiments in presence of additional substances.

In summary, the present inventors have found that a combination of linseed oil and castor oil ranging from 70% linseed oil and 30% castor oil to 85% linseed oil and 15% castor oil, preferably 75% linseed oil and 25% castor oil, show an improved profile compared to commercially available oils in terms of cell homeostasis, absorption and reactive oxygen species production. In addition the importance of nanoemulsion to improve its biological function was confirmed.

### Example 2

*Effects of Vitamin D3 dissolved on vegetable oils both on in vivo and in vitro study:* To investigate the effect of Vitamin D3 (VD3) dissolved in vegetable oils, namely linseed oil and castor oil, the cell viability of Huh-7 and GTL-16 cells was investigated by MTT test, and compared to the effect of VD3 dissolved in ethanol and in olive oil, solvents conventionally used to dissolve VD3. Further, the efficacy of VD3 dissolved in vegetable oils was compared with commercially available VD3 (Cholecalciferol 0.625mg, equivalent to 25.000 I.U., 2.5 ml solution for oral administration; indicated herein as "VD3 DIBASE").

Fig.s1A (Huh-7) and 1B (GTL-16) show that VD3 dissolved in pure linseed oil (O1) and castor oil (O2) have similar effect when compared to VD3 solubilized in ethanol, as well as VD3 dissolved in olive oil (O3) and commercial VD3 (V di base), in both cell lines. All data were statistically significant compared to control (p<0.05).

Fig.s1D and 1E show the effect on cell viability of VD3 solubilized in a mixture of linseed oil and castor oil. As shown in Fig.1C and described in Example 1, a range of optimal concentrations of linseed oil and castor oil was identified. VD3 dissolved in different percentages of linseed oil and castor oil was used to perform an MTT test on Huh-7 and GTL-16 cells. Surprisingly, VD3 dissolved in 75% linseed oil/25% castor oil mixture (VD3_75%O1/25%O2) had the maximum effect on cell viability in both cell lines (Huh7 65±3% vs control; GTL-16 47.31±2.08% vs control; p<0.05), also in comparison with VD3 dissolved in Ethanol and commercially available VD3 (Huh7 36±2.8% vs control; GTL-16 36.67±1.53% vs control; p<0.05).

The ability of VD3 to improve cell viability is a well known test of its bioactivity and correlates with its in vivo efficacy. Hence, VD3 dissolved in 75% linseed oil/25% castor oil mixture is demonstrated to have a better efficacy profile compared to available current formulations for human use.

Since all these data supported a new formulation of VD3 with 75% linseed oil/25% castor oil mixture, some additional experiments have been carried out in order to verify the effectiveness of 75% linseed oil/25% castor oil nanoemulsioned mixture on stomach and intestinal cells. As reported in Fig.7A, VD3 with 75% linseed oil/25% castor oil mixture prepared as a nanoemulsion, was able to increase cell viability on both cell type (p<0.05) compared to control and the same preparation without nanoemulsion (p<0.05). These data confirmed the importance of nanoemulsion during preparation. Since the effects of VD3 are obtained only if its intracellular concentration increases, the inventors checked the intracellular concentration of VD3 prepared with 75% linseed oil/25% castor oil mixture with or without nanoemulsion on both cell type (Fig.7B). This is an important parameter about the rate absorption and relative delivered of VD3 after oral intake. VD3 prepared with 75% linseed oil/25% castor oil mixture without nanoemulsion was able the increase its intracellular concentration on both cell type comparing to control (p<0.05) and to VD3 prepared in ethanol (p<0.05) or to commercial VD (p<0.05). VD3 prepared with 75% linseed oil/25% castor oil mixture with nanoemulsion was able to further increase the intracellular concentration of VD also compared to control, VD3 prepared in ethanol, commercial VD (p<0.05) and also to the same preparation without nanoemulsion (p<0.05) on GTL-16 and Caco-2 cells. These data are important because demonstrate the ability of 75% linseed oil/25% castor oil mixture to improve the absorption of VD and the importance of nanoemulsion to delivered the substances dissolved in the mixture oils. Moreover, to investigate the impact of 75% linseed oil/25% castor oil mixture with or without nanoemulsion on ROS and MDA production on both cell types, additional experiments were performed. VD3 in 75% linseed oil/25% castor oil mixture without nanoemulsion was able to reduce the ROS production (Fig.7C) compared to commercial VD (p<0.05) on both cell type and the same preparation with nanoemulsion amplified this effect (p<0.05). In addition VD3 in 75% linseed oil/25% castor oil mixture with nanoemulsion induced a less ROS release (p<0.05) compared to the sample preparation without nanoemulsion. VD3 in 75% linseed oil/25% castor oil mixture without nanoemulsion was able to reduce the MDA production (Fig.7D) compared to commercial VD (p<0.05) on both cell type and the same preparation with nanoemulsion amplified this effect (p<0.05). In addition VD3 in 75% linseed oil/25% castor oil mixture with nanoemulsion induced a less MDA production (p<0.05) compared to the sample preparation without nanoemulsion. These confirmed the beneficial effects of VD on 75% linseed oil/25% castor oil mixture on stomach and intestine and the importance of nanoemulsion to support the effect of VD3. As the biological effects of VD3 reported before, may also involve the VDR receptor, additional experiments were carried out to analyze the involvement of VDR in stomach and intestine. As reported in Fig.s7E and F, the expression of VDR receptor was increased after the treatment with VD3 in 75% linseed oil/25% castor oil mixture on GTL-16 and Caco-2 cells compared to control (p<0.05) and to commercial VD. In addition the same preparation of VD3 in 75% linseed oil/25% castor oil mixture with nanoemulsion, induced a major increased than control, commercial VD and the same preparation without emulsion (p<0.05). These data confirmed the involvement of VDR receptor in the mechanism activated by VD3 in 75% linseed oil/25% castor oil mixture and the importance of emulsion to maintain more effectiveness VD after the administration.

The physiological reactions of the cells to treatment with different formulations of VD3 were also measured analyzing ROS production in both Huh-7 and GTL-16 cell lines by a colorimetric assay based on cytochrome C reduction. This parameter is important to determine the health of the tissue after the treatment, since an excess of oxidative stress can lead to various pathological conditions.

Fig.s3A (Huh-7) and 3C (GTL-16) show the effect on ROS production of VD3 dissolved in pure vegetable oils. Each solvent tested induced a statistically significant increase of ROS release (p<0.05), and the presence of VD3 was able to reduce ROS release, compared to the vegetable oils alone, with a maximum effect when dissolved in pure linseed oil (Huh7 21±3.89 nmol/µg of protein; GTL-16 23.33±1.53 nmol/µg of protein; p<0.05).

Fig.s3B (Huh-7) and 3D (GTL-16) show the effect on ROS production of VD3 dissolved in mixed vegetable oils. VD3 dissolved in 75% linseed oil/25% castor oil mixture induced the highest reduction of ROS release when compared to the composition of pure vegetable oils alone (Huh7 16.08±2.94 nmol/µg of protein; GTL-16 19.4±2.02 nmol/µg of protein; p<0.05), as well as commercially available VD3 (VD3 DIBASE) indicating a better efficacy of the formulation, on both cell types, demonstrating that the mixed oil formulation has the advantageous effect of reducing oxidative stress.

Cell proliferation is considered a well known marker of VD3 bioactivity. The ability to proliferate of Huh-7 treated with various VD3 formulations was investigated by crystal violet assay, which allows a cell count after the treatment, as well as by KI67 protein Western blot and densitometric analysis. KI67 is a well known nuclear protein marker of proliferating cells.

As reported in Fig.4A, VD3 formulated in pure vegetable oils induced an increased cell proliferation (p<0.05) compared with solvents alone. The maximal effect was observed with VD3 formulated in the 75% linseed oil/25% castor oil mixture (enhancement of 48% vs control; p<0.05) and this effect was statistically significant compared to VD3 dissolved in pure oil as well as to commercial VD3 (VD3 DIBASE; enhancement of 35.3% vs VD3 DIBASE; p<0.05).

These results confirmed the beneficial role of the new VD3 formulation on cell proliferation without increase of oxidative stress.

As shown in Fig.4B, KI67 protein levels were analysed by Western blot analysis in Huh-7 cells treated with VD3 formulated in the 75% linseed oil/25% castor oil mixture. The75% linseed oil/25% castor oil solvent mixture alone did not increase Ki67 levels, but the presence of VD3 induced a significant increase of Ki67 expression (80% increase) compared to the negative control and to the commercial VD (VD3 DIBASE, 60% increase), confirming the findings reported above regarding the enhanced bioactivity of the new VD3 formulation.

One of the intracellular pathways activated by VD3 is the ERK^{1/2}/MAPK pathway, involved in cell migration and survival. VD3 has been shown to induce fast non-transcriptional responses involving stimulation of transmembrane signal transduction pathways and activate the extracellular signal-regulated mitogen-activated protein (MAP) kinase, in particular the ERK¹ and ERK² isoforms. Said pathway activation was therefore analyzed by Western blot and densitometric analysis as means to quantitate VD3 bioactivity.

As shown in Fig.s5A and 5B, Western blot and densitometric analysis respectively demonstrated that among the pure oils, castor oil induced increased phosphorylation of ERK^{1/2}, indicating a possible lipidic stress of the cells (p<0.05), given the fact that ROS production was also increased. However, VD3 dissolved in the same oil caused an increase of the ERK^{1/2} phosphorylation within a physiological range and in presence of castor oil the negative effects of the oil alone were reduced. Pure linseed oil did not increase ERK^{1/2} phosphorylation, and the presence of VD3 induced the expected increase in phosphorylation of ERK^{1/2} demonstrating that the effect on phosphorylation is purely mediated by VD3 and not by the solvent.

Mixing linseed oil and castor oil quenches the effect of castor oil alone.

Hence, VD3 formulated in the 75% linseed oil/25% castor oil mixture is able to induce beneficial effects without the induction of oxidative stress, a condition that occurs when the cells are stimulated by the oil alone. For this reason, the novel VD3 formulation improves VD3 beneficial effects.

Furthermore, the activation of p53, a marker of apoptosis, was evaluated in the experimental conditions described above, by Western blot and densitometric analysis on Huh-7 cells.

As reported in Fig.s5A and 5B, castor oil alone caused a significant increase (p<0.05) in p53 activation compared with control and with linseed oil alone. The presence of VD3 reduced this activation, indicating the efficacy of VD3 to prevent cell cycle damage. On the contrary, linseed oil alone or a 75% linseed oil/25% castor oil mixture did not activate p53 and VD3 amplified its effects (p<0.05).

It is noteworthy that the commercial VD3 (VD3 DIBASE, prepared in refined olive oil) caused activation of p53 compared to control, contrary to VD3 formulated in the 75% linseed oil/25% castor oil mixture, confirming the positive effects of this combination on cellular function.

The level of expression of the VDR receptor is a commonly used marker for VD3 activation, since a positive feedback loop increases VDR expression levels upon engagement of the receptor to its ligand. As described in Fig.s5A and 5, the vegetable oils pure or mixed did not induce significant changes of VDR expression, as measured by Western blot analysis whilst the presence of VD3 enhanced VDR expression. Notably, the maximum effect was observed when VD3 was dissolved in the 75% linseed oil/25% castor oil mixture (54.7±3.52% vs control). The expression was also statistically different compared to commercial Vitamin D VD3 DIBASE; 21.03±2.7% vs control).

The data above demonstrate an improved profile of VD3 formulated in the 75% linseed oil/25% castor oil mixture on cellular functions, with a better influence on cellular integrity, and an optimized anti oxidative effect on tissues expressing the VDR, when compared to commercially available VD3 as well as other formulations of VD3.

Since VD3 is well known to exert its beneficial effects on ovarian and cardiac cells, some additional experiments with 75% linseed oil/25% castor oil nanoemulsion mixture were performed. Vitamin D receptor (VDR) and the enzymes of its metabolism have been found in both male and female reproductive tissues: there is evidence that vitamin D is involved in female reproduction, including in vitro fertilization and polycystic ovary syndrome. In women with polycystic ovary syndrome, it is a lower plasma concentration of VD3. These levels are generally associated with obesity and metabolic syndrome.

CHO cells (Chinese hamster ovary) are an epithelial cell line derived from the ovary of the Chinese hamster, often used in biological and medical research The H9c2 cell line was originally derived from embryonic rat ventricular tissue and they still show many similarities to primary cardiomyocytes, including membrane morphology, signalling protein expression and electrophysiological properties. VD3 exert a protective role on sudden cardiac death. In addition, VD3 deficiencies cause a myocardial dysfunction and cardiac failure. VD3 play also a role on the regulation of blood pressure: low levels of VD3 are associated with increased risk of hypertension. For these reasons, the inventors would to test VD3 on ovarian and cardiac cells. As reported in Fig.8A on CHO cells, nanoemulsion 75% linseed oil/25% castor oil mixture alone was able to increase cell viability compared to control (p<0.05) confirming the beneficial effects of the oils on ovarian cells hypothesizing a systemic beneficial effect of the mixture. VD3 with nanoemulsion 75% linseed oil/25% castor oil mixture was able to enhance the cell viability compared to control, and commercial VD (p<0.05), indicating a better influence of VD3 with nanoemulsion 75% linseed oil/25% castor oil mixture on cell function on ovarian tissue. Similarly on cardiac cells, 75% linseed oil/25% castor oil mixture induced an increase on cell viability compared to control (p<0.05) and this effect was amplified by the presence of VD3 (p<0.05). In addition VD3 with nanoemulsion 75% linseed oil/25% castor oil mixture was able to induce an improvement on cell viability compared to the commercial VD (p<0.05). To demonstrate the better influence on cellular function of VD3 with nanoemulsion 75% linseed oil/25% castor oil mixture, the same treatments were conducted to analyze ROS production on CHO cells (Fig.8B). The nanoemulsion 75% linseed oil/25% castor oil mixture didn't cause an enhancement on ROS release compared to control (p>0.05), indicating a tolerability of the cell to the oils mixture. VD3 with nanoemulsion 75% linseed oil/25% castor oil mixture on the other was able to reduce the ROS production compared to control and to commercial VD (p<0.05), indicating a beneficial effect and confirming the antioxidant effect of the molecules. In addition on H9c2 cells, the nanoemulsion 75% linseed oil/25% castor oil mixture caused a reduction on ROS release compared to control (p>0.05), and the presence of VD3 didn't caused a significant changes on cell viability. VD3 with nanoemulsion 75% linseed oil/25% castor oil mixture was also able to reduce the ROS production compared to commercial VD (p<0.05), confirming its beneficial effects.

Since the ability of 75% linseed oil/25% castor oil mixture and VD3 with nanoemulsion 75% linseed oil/25% castor oil mixture it should also be demonstrated in a complex biological system, the inventors decided to test these solutions on rat animal model, measuring vasodilation and nitric oxide release (NO). NO is produced by vascular endothelial cells in response to different stimuli and acts as a messenger molecule, activating guanylate cyclase to enhance cGMP which in turn causes relaxation of smooth muscle and vasodilation. NO is produced by nitric oxide synthases (NOS) and the endothelial isoform of NOS (eNOS) is expressed constitutively in endothelial cells and it is responsible for the basal release of nitric oxide from the endothelium and for the rapid change in nitric oxide flux in response to physical stimuli and molecular agonists. The impaired NO status may cause an overproduction of reactive oxygen species (ROS) in the vasculature. As reported in Fig.9A, after the intravenous infusion at 120 sec the aortic blood flow on the rats treated with nanoemulsion 75% linseed oil/25% castor oil mixture was augmented compared to control (p<0.05) and with VD3 this increase was amplified compared to the mixture oils alone (p<0.05) and this increase was major then commercial VD (p<0.05). To understand the influence on vasodilation, in the same condition was verify the NO release on plasma rats. As reported in Fig.9B, VD3 75% linseed oil/25% castor oil mixture induce a significant NO release compared to control and commercial VD (p<0.05) supporting the data observed on vasodilation.

The data above demonstrate an improved profile of VD3 formulated in the nanoemulsion 75% linseed oil/25% castor oil mixture on cellular functions of ovarian and cardiac tissues, with an optimized antioxidative effect when compared to commercially available VD3. Finally the important findings obtained from in vivo experiments, demonstrated the applicability of the invention and the results can be transferred to human basing on translationality between the experimental study and human application.

### Example 3

*Stability of Vitamin D formulated in vegetable oils:* Vitamin D in liquid form is susceptible of oxidation upon exposure to air and light. Hence, vials containing Vitamin D are made with dark glass and are kept refrigerated, in the dark, once open even upon taking said precautions; the bioactivity of VD formulations is limited in time. The present inventors examined the stability of different preparations of vitamin D at 4°C. The effect of VD3 dissolved in linseed oil 100% (V in O1 100%), in castor oil 100% (V in O2 100%), in olive oil (V in O3 100%) and in 75% linseed oil with 25% castor oil (V in 75%) compared to a commercial vitamin D preparation (VD3 DIBASE) were tested in Huh7 cells. Cell viability was measured by MTT test and ROS production was measured by assessment of cytochrome c reduced per mg of protein. Once open, the chemical stability during time of the commercial vitamin D (VD3 DIBASE) is guaranteed for 5 months. After this time, the bioactivity is reduced by oxidation. Therefore, we have tested the efficacy of the different formulations at different time points, after opening, from 1 to 8 months. As reported Fig.10 the cell viability of V in 75% is significantly higher than what observed in other formulations during the first 5 months, and for a longer period of time.

Since oxidation of vegetable oils is a natural process during time, detection of some ROS production it is normal. As reported in Fig.10 the level of ROS production is time-dependent for all preparations, and V in 75% induced a production similar to V in 100% linseed oil. However, both VD3 in pure linseed oil and VD3 in 75% linseed oil/25% castor oil mixture showed lower levels of ROS production when compared to VD3 in pure olive oil, in castor oil and the commercial VD3 preparation, VD3 DIBASE, which uses refined oil as solvent.

In conclusion the formulation of VD3 in 75% linseed oil/25% castor oil mixture (V in 75%) preserved its beneficial properties over time and the oxidative species naturally produced were less than the other tested formulations including the commercial vitamin D (VD3 DIBASE).

### Example 4

*Effects of Resveratrol, Q10 and Melatonin dissolved on emulsion vegetable oils with or without Vitamin D3 on in vivo and in vitro study:* To demonstrate that nanoemulsion of oils mixture are efficient also with other lipophilic agents, additional experiments were performed in vitro on GTL-16, CHO, H9c2 and Caco-2 cell lines and in vivo model through intravenous injection in rats. Resveratrol (R), Q10 and Melatonin (M) were used at 50µM for 2 minutes. All these concentrations used are in a physiological range and hypothesized to use in human, basing on FDA conversion animal-human.

R is a natural polyphenol synthesized by several plants as a phytoalexin when under attack by pathogens such as bacteria or fungi. This bioflavonoid is naturally found in mulberries, peanuts, several species of medicinal plants and is present in the skin of grapes and thus in red wine. R possesses a broad spectrum of pharmacological and therapeutic properties and recently some studies have explained the role of R in such vital biological functions as reproduction and ovarian function.

Later studies on Q10 demonstrated its presence in other subcellular fractions and in plasma, and extensively investigated is its antioxidant role. Q10 is the only lipid soluble antioxidant synthesized endogenously. Because of its hydrophobicity and large molecular weight, absorption of dietary CoQ10 is slow and limited. It has a direct anti-atherogenic effect, which has been demonstrated in apolipoprotein E-deficient mice fed with a high fat diet. Animal data show that Q10 in large doses is taken up by all tissues including heart and brain mitochondria. This has implications for therapeutic applications in human diseases, and there is evidence for its beneficial effect in cardiovascular and neurodegenerative diseases.

M is found in humans, animals, plants and microbes and it is a lipophilic compound diffusing rapidly through biological membranes and is involved in many regulatory processes, such as biological rhythms, intestinal reflexes, and protection against inflammation, metabolism and reproduction. The amount of melatonin in the digestive system is 400 fold greater than the pineal gland. M is produced in specialized cells called enteroendocrine cells of the gastrointestinal tract. This super hormone is present in all segments of the gastrointestinal tract. M is involved in the regulation of multiple functions, including the control of the gastrointestinal system under physiological and pathophysiological conditions. M is also an important regulator of both inflammation and motility in the gastrointestinal tract.

The effects of R, Q10 alone or in combination with VD3 on cellular function, such as cell viability, Radical Oxygen Productions (ROS), was analyzed in GTL-16, Caco-2, CHO and H9c2 cell lines. GTL-16 cell line is an epithelial gastric carcinoma cell line widely used to examine tolerability of human gastric mucosa in presence of vegetable oils and other substances. Caco-2 cells are a human colon epithelial cancer cell line used as a model of human intestinal absorption of drugs and other compounds and are applied to predict the intestinal absorption following oral exposure. CHO cells are an epithelial cell line derived from the ovary of the Chinese hamster, often used in biological and medical research. The H9c2 cell line was originally derived from embryonic rat ventricular tissue and they still show many similarities to primary cardiomyocytes.

In Fig.8A on GTL-16 cells, R with nanoemulsion 75% linseed oil/25% castor oil mixture and Q10 with nanoemulsion 75% linseed oil/25% castor oil mixture were able to enhance the effect of R and Q10 without mixed oils (p<0.05). The presence of VD3 with R or Q10 with nanoemulsion 75% linseed oil/25% castor oil mixture amplified its effects (p<0.05). These data confirmed the beneficial effects exerted by mixture oils alone and in presence of VD3 on antioxidant substances. In addition, these data supported the ability to act via nongenomic pathway.

Since R was known to act on gynecological disorders, some experiments was performed on ovarian cells. As reported in Fig.8A, R with nanoemulsion 75% linseed oil/25% castor oil mixture was able to improve the cell viability of CHO cells compared to control (p<0.05) and to R in absence of mixed oils (p<0.05). In addition this effect was more evident in the samples treated with R and VD3 adding together with nanoemulsion 75% linseed oil/25% castor oil mixture (p<0.05). These findings confirmed the importance of R in nanoemulsion mixed oils on ovarian tissue. Another important tissue well know to be involved by the effect of the antioxidant was cardiac tissue, for this reason the inventors tested the ability of Q10 prepared in nanoemulsion 75% linseed oil/25% castor oil mixture on H9c2 cells (Fig.8A). The presence of nanoemulsion 75% linseed oil/25% castor oil mixture with Q10 caused a significant increased (p<0.05) of cell viability compared to control and to Q10 prepared in absence of mixture oils. In addition this effect was amplified (p<0.05) by the presence of VD3 with Q10 prepared in nanoemulsion 75% linseed oil/25% castor oil mixture. These data confirmed the importance of Q10 on cardiac function and demonstrate the ability of nanoemulsion mixed oils to improve its beneficial effects. Additional experiments were performed to investigate the impact of R and Q10 on ROS (8B) and Malondialdehyde (MDA) production (8C) in GTL-16, CHO and H9c2 cell lines. In GTL-16 cell lines R in nanoemulsion 75% linseed oil/25% castor oil mixture caused a significant changes on ROS production compared to control indicating that the antioxidant property of R was maintained (Fig.8B). In addition the combination of R in nanoemulsion 75% linseed oil/25% castor oil mixture and VD3 in 75% linseed oil/25% castor oil mixture was able to reduce the ROS production compared to control (p<0.05) and to the cells treated only with R in nanoemulsion 75% linseed oil/25% castor oil mixture, confirming the ability of VD3 to improve the beneficial effect of R. In GTL-16, the inventors tested also Q10 prepared with nanoemulsion 75% linseed oil/25% castor oil mixture in presence or absence of VD3 (Fig.8B) to analyzed ROS release. In these cells Q10 and VD3 in nanoemulsion 75% linseed oil/25% castor oil mixture was able to maintained the ROS production at physiological level and a greater effect was shown compared to Q10 in nanoemulsion 75% linseed oil/25% castor oil mixture alone. Finally the importance of the solubilization of R and Q10 on GTL-16 was confirmed by the results obtained from MDA release (Fig.8C), in which all did not caused any significant changes between the different solution, indicating the maintenance of the physiological level of radicals after stimulation without damage in the tissue.

Since R and Q10 acts primarily at ovarian and cardiac tissue, some additional experiments would be performed to explain the effect of R and Q on ROS release.

In CHO cell lines R in nanoemulsion 75% linseed oil/25% castor oil mixture was able to statistically improve (p<0.05) its antiradical property compared to control and the R without mixed oils (Fig.8B). In addition, the presence of VD3 was able to reduce the ROS production compared to control (p<0.05) and to R in nanoemulsion 75% linseed oil/25% castor oil mixture alone (p<0.05), confirming the ability of R to act on ovarian oxidative condition. To define the ability of R in nanoemulsion 75% linseed oil/25% castor oil mixture also in the absorption during the treatments and to hypothesized its possible absorption rate after oral intake, in Fig.8D was shown the intracellular concentration of trans-resveratrol (trans-R), the main metabolite derived from R, measured by HPLC, in CHO cells. R in nanoemulsion 75% linseed oil/25% castor oil mixture already in 2 min was able to cross the cellular membrane and then it is converted to trans-R. From R in nanoemulsion 75% linseed oil/25% castor oil mixture the inventors observed a significant increase on trans-R compared to control and compared to R without mixture (p<0.05). In addition this effect when VD3 was added to R in nanoemulsion 75% linseed oil/25% castor oil mixture was better than control and the same stimulation without VD3 (p<0.05). All these results confirmed the ability of R to cross the cellular membrane before to exert its beneficial effects that they depend from the concentration of trans-R present in the ovarian tissue. In addition, these data demonstrates the importance of mixed oils to deliver the substances to cross intracellular membrane.

In H9c2 cell lines (Fig.8B), Q10 in nanoemulsion 75% linseed oil/25% castor oil mixture maintained at basal level the ROS production, confirming its ability to protect from ROS damage and with VD3 this effect was greater than the effect obtained using Q10 in nanoemulsion 75% linseed oil/25% castor oil mixture alone (p<0.05). These data demonstrated the improvement of nanoemulsion 75% linseed oil/25% castor oil mixture on Q10 to exert its effects.

To demonstrate the effectiveness of Q10 on cardiac function, the inventors decided to test Q10 in nanoemulsion 75% linseed oil/25% castor oil mixture alone with or without VD3 in rats, measuring vasodilation and Nitric Oxide release (NO) after intravenous injection. As reported in Fig.9A, Q10 caused a significant vasodilation compared to control (p<0.05) and in present of nanoemulsion 75% linseed oil/25% castor oil mixture this effect was more evident (p<0.05) after 120sec of infusion. In addition, Q10 in nanoemulsion 75% linseed oil/25% castor oil mixture with VD3 amplified the effect observed without VD3 (p<0.05). To explain the mechanism involved in vasodilation one important parameter of vasodilation, NO release, was measured on plasma sample at 120sec. The data observed confirmed and support a previous finding about vasodilation (Fig.9B); indeed Q10 caused an increase on NO release compared to control (p<0.05) and this effect was amplified using Q10 in nanoemulsion 75% linseed oil/25% castor oil mixture (p<0.05). In addition, the presence of VD3 with Q10 in nanoemulsion 75% linseed oil/25% castor oil mixture caused the maximum NO release (p<0.05).

These findings demonstrated the applicability of Q10 in nanoemulsion 75% linseed oil/25% castor oil mixture on the animal model and the results can be transferred to human basing on translationality between the experimental study and human application.

Since the molecules insoluble or poorly soluble in water have a different solubility rate, the inventors have decided to check the best conditions to solubilize maintaining the effectiveness of M on GTL-16 and Caco-2 cells. The cell viability of GTL-16 (Fig.11A) and Caco-2 (Fig.11B) was differently influenced by M in nanoemulsioned mixed oils and the range that caused the most increase are from 75% to 85% and from 75% to 90% respectively, compared to control (p<0.05). The better results on both cell types was observed using M in nanoemulsion 80% linseed oil/20% castor oil mixture after 2min of stimulation. This concentration was maintained in all successive experiments on both cell types. Since another important parameter regarding the mechanism activated by M was ROS production, additional experiments were performed on GTL-16 and Caco-2 cells (Fig.11C). M in nanoemulsion 80% linseed oil/20% castor oil mixture on GTL-16 was able to maintain the radical production at basal level and when VD3 was added together with M in nanoemulsion 80% linseed oil/20% castor oil mixture this production was decreased. In addition on intestinal cells, M in nanoemulsion 80% linseed oil/20% castor oil mixture caused a significant reduction (p<0.05) on ROS release compared to control and the presence of VDR amplified the effect (p<0.05). These data confirming the ability of M to act preventing the radical release after oral intake. M exerts its physiological effects through specific membrane receptors, named melatonin-1 receptor (MT1), MT2 and MT3. These receptors can be found in the gut and their involvement in the regulation of GI motility, inflammation and pain has been reported in numerous basic and clinical studies. For this reason, the inventors decided to investigate the involvement of MT1 on both cell types. M in nanoemulsion 80% linseed oil/20% castor oil mixture caused a significant increase (p<0.05) of MT1 activation
on both cell types compared to control, as reported by Western blot (Fig.11E) and densitometric analysis (Fig.11D). In addition these effects were amplified by the presence of VD3 (p<0.05), confirming the importance of VD3 to support the effect of M. Summarizing these data confirm the importance of solubilization of the molecules in order that they can play and keep their biological properties. These data also confirmed the potential use of the VD3 in combination with other compounds.

### Example 5

The present inventors examined the protective role of VD3 on oxidative stress, particularly upon H2O2 treatment.

ASA is a well known anti inflammatory drug which exerts its anti-inflammatory effects through multiple mechanisms of action that include generation of reactive oxygen species (ROS), and increase of oxidative stress. Many pathologies of stomach and liver are caused by oxidative injury, via ROS or nitric oxide, and adverse effects observed in liver and stomach by long term pharmacological treatment with the same drugs (such as ASA) have been linked to increase of the oxidative stress condition. Among the non classical mechanisms of action for Vitamin D3, such as apoptosis inhibition and improvement of autophagy and survival mechanisms, prevention of oxidative damage in various tissues has been demonstrated.

The present inventors have surprisingly found that VD3 when dissolved in a 75% linseed oil/25% castor oil mixture improves cell survival upon oxidative stress, and has a protective role on cells treated with ASA, by decreasing ROS production. *Effects of VD3 in cells treated with ASA, before or after oxidative stress:* Huh-7 and GTL-16 cells have been treated with different concentrations of ASA (0.05-20µmol/ml) alone or in combination with 10µM VD3 dissolved in a 75% linseed oil/25% castor oil mixture (abbreviated as VD3), to study the effects on cell viability in presence or absence of oxidative stress induced by 200µM H₂O₂. The results show a statistically significant dose dependent decrease (p<0.05) on cell viability of both cell types in presence of different concentrations of ASA. The co-treatment of the cells with ASA and VD3 modulated the negative effects of ASA on both cell types, and 10µM VD3 in presence of 0.05µmol/ml ASA was able to induce a significant increase on cell viability in GTL-16 cells (12.31±0.6% vs control). These data demonstrated the ability of VD3 to counteract the negative effects on cell viability of ASA. In addition, the presence of oxidative stress induced by the treatment with 200µM H₂O₂ for 30 minutes caused a significant decrease on cell viability (p<0.05) in both cell types. This condition has been also tested before and after the stimulation with ASA alone or in presence of VD3. The oxidative stress induced before or after the stimulation with ASA alone caused a reduction of cell viability, whilst the presence of VD3 has been able to counteract in a statistically significant manner these negative effects (p<0.05); in particular, the most beneficial effects of VD3 have been observed in presence of ASA 0.05µmol/ml in both cell types. Since oxidative stress causes an increase of ROS production, ROS release was measured in both Huh-7 and GTL-16 cells. The results show that in both cell types the different doses of ASA (0.05-20µmol/ml) induced a dose-dependent ROS release which was reduced upon co-treatment with VD3 (p<0.05). Moreover, the treatment for 30 minutes with 200µM H₂O₂ causes a significant increase of ROS production (p<0.05;) in both cell types compared to control (Huh7 24.54±1.89 nmol/µg of protein; GTL-16 34.86±1.79 nmol/µg of protein; p<0.05).

Finally the applicant has found that the treatment with 200µM H₂O₂ for 24hr before the stimulation with different concentrations of ASA alone, induces an increase of ROS release which was greater than upon treatment with ASA alone or with 200µM H₂O₂ alone, more evident with ASA 20µmol/ml in both cells types (Huh7 50±1.41 nmol/µg of protein; GTL-16 49.19±1.45 nmol/µg of protein; p<0.05).

VD3 is able to counteract in a statistically significant manner the ROS release in both cell types (p<0.05). In particular, these effects were more evident in Huh-7 cells. The treatment with 200µM H₂O₂ after the stimulation with different concentrations of ASA alone causes more ROS production in both Huh-7 and GTL-16 cells (p<0.05), compared to the experimental condition where the cells were pre-treated with H₂O₂, and the presence of VD3 was able to prevent this effect, which was more evident with ASA 20µmol/ml (Huh7 16.46±0.11 nmol/µg of protein; GTL-16 18.36±2.72 nmol/µg of protein; p<0.05).

*Analysis of anti-proliferative activity of ASA and role of Vitamin D:* In order to further investigate VD3 protective role on cellular damage caused by ASA treatment, the expression of Annexin V has been evaluated. Annexin V is a protein marker for membrane translocation of phosphatidilserines from the cytoplasm which is a sign of cellular toxicity. Hence, the presence of Annexin V in the same experimental conditions as above has been analyzed by immunocytochemistry. Furthermore, P53 activation has been assessed, since a critical role for tumor suppressor genes in the cellular response against oxidative stress has been recently identified.

The appearance of phosphatidylserine residues (normally hidden within the plasma membrane) on the surface of the cell has been detected in presence of different doses of ASA alone (0.05-20µmol/ml), and this effect was reduced in presence of VD3 (p<0.05); this is more evident in presence of 20µmol/ml ASA (reduction of 54%). When the cells has been treated with 200µM H₂O₂ after ASA treatment (0.05-20µmol/ml), the number of positive cells increases compared to ASA treatment alone (p<0.05), indicating a progressive damage caused by oxidative stress. In the samples treated with 200µM H₂O₂ after the stimulation with ASA (0.05-20µmol/ml), the effects were similar to what described in the sample pre-treated with 200µM H₂O₂. When the cells have been pre-treated with 200µM H₂O₂, this damage is more evident. In both experimental conditions, i.e. H₂O₂ administration before or after ASA treatment, the presence of VD3 is able to modulate the translocation of phosphatidylserine that is recognized by Annexin V immunostaining, indicating the ability of VD3 to modulate the mediators of oxidative stress and the negative effects of ASA on the cells. The densitometric analysis of p53 (confirms the effects observed with Annexin V staining: in particular, VD3 is able to reduce the activation of p53 to a basal level when the cells have been treated with ASA only. In presence of oxidative stress (before or after stimulation with ASA), the treatment with VD3 is able to maintain the activation of p53 at a lower level than the oxidative stress alone.

## Claims

1. An edible composition comprising castor oil and linseed oil, **characterized in that** the castor oil: linseed oil volumetric ratio is 25:75

2. The composition according to claim 1 further comprising at least one first additional ingredient selected from the list of: lipophilic vitamins, preferably selected from: Vitamin A, Vitamin D, Vitamin E and Vitamin K, even more preferably Vitamin D3; poliphenols, preferably selected from: anthocyanins, curcumin, resveratrol, sylmarin, and quercetin; flavonoids; bioflavonoids; phytoestrogens, preferably an isoflavon, preferably selected from: genistein, daidzein and glycitein; carotenoids, preferably selected from: astaxanthin, beta-carotene, crocetin, lycopene, lutein, and zeaxanthin; serenoa repens extracts; PDE5 inhibitors; Coenzyme Q10; and melatonin.

3. The composition of claim 2, wherein the amount Vitamin D3 lies from 5µg/ml to 125 µg/ml and from 400IU to 5000IU.

4. The composition according to anyone of claims 1-3, further comprising a second additional ingredient selected from the list of acetylsalicylic acid, lipophilic vitamins, polyphenols, flavonoids, bioflavonoids, Coenzyme Q10, melatonin and metformin, preferably this second additional ingredient being acetylsalicylic acid.

5. The composition of claim 4, wherein the amount of acetylsalicylic acid ranges from 0.05µmol/ml to 20µmol/ml or from 10 µg to 650 µg.

6. A process for preparing a nanoemulsion comprising the composition according to anyone of claims 1-5, said process comprising at least one step, preferably 1-10 steps, more preferably 2-5 steps, of homogenizing the composition of castor and linseed oils, wherein linseed oil is preferably added before castor oil, in the ratio according to claim 1 under cooling condition, for 1-5 minutes, preferably for 1-2 minutes, for each homogenizing step wherein said homogenization is performed at 1000-5000rpm, preferably at 1500-3000rpm, more preferably at about 2000rpm.

7. The process according to claim 6 wherein the composition comprises further a first additional ingredient according to anyone of claims 2 or 3 and/or a second additional ingredient according to anyone of claims 4 or 5.

8. The process according to anyone of claims 6 or 7, wherein the nanoemulsion is solidified, preferably at room temperature, after being added in drops to a solution of gelatin or maltodextrin wherein said solution of gelatin or maltodextrin is at a concentration of 10-30%, preferably about 20%, or the nanoemulsion being solified by dry congealing or silica gel.

9. A nanoemulsion obtained by using the process according to anyone of claims 6-8.

10. The composition according to anyone of claims 1-5, or the nanoemulsion according to claim 9 for use as medicament, preferably as an antipyretic or analgesic agent.

11. The composition according to anyone of claims 1-5, or the nanoemulsion according to claim 9 for use in the treatment of a disease selected from: a cardiac disease, stomach ulcer, liver damage, stroke, kidney insufficiency, inflammatory disease preferably selected from: ankylosing spondylitis, osteoarthritis, rheumatoid arthritis, Systemic Lupus Erythematosus (SLE), SLE-associated arthritis and SLE-associated pleurisy, or rheumatic fever, myocardial infarction, and ischemic stroke; and malabsorption syndrome, or for use in the prophylaxis of thromboembolic stroke or revascularization procedures.

12. The compositions of claims 4 or 5 for use in the treatment of kidney failure, hemorrhagic lesions, gastric ulcer, hemostasis disease, cardiovascular stroke, brain stroke, liver insufficiency, iatrogenic disease or Reye syndrome.

13. The compositions according to claims 1-5 for use in the treatment of patients undergoing anti-clotting therapy, anti-thrombosis therapy, post cardiac surgery, or a post trauma therapy.

14. Use of a composition according to anyone of claims 1-5, or the nanoemulsion according to claim 9 as nutraceuticals, dietary supplements or food supplements.

## Patentansprüche

1. Essbare Zusammensetzung, umfassend Rizinusöl und Leinöl, **dadurch gekennzeichnet, dass** das Volumenverhältnis Rizinusöl:Leinöl 25:75 beträgt

2. Zusammensetzung nach Anspruch 1, ferner umfassend mindestens einen ersten zusätzlichen Inhaltsstoff ausgewählt aus der Liste von: lipophilen Vitaminen, vorzugsweise ausgewählt aus: Vitamin A, Vitamin D, Vitamin E und Vitamin K, noch bevorzugter Vitamin D3; Poliphenolen, vorzugsweise ausgewählt aus: Anthocyanen, Curcumin, Resveratrol, Sylmarin und Quercetin; Flavonoiden; Bioflavonoiden; Phytoöstrogenen, vorzugsweise einem Isoflavon, vorzugsweise ausgewählt aus: Genistein, Daidzein und Glycitein; Carotinoiden, vorzugsweise ausgewählt aus: Astaxanthin, Beta-Carotin, Crocetin, Lycopin, Lutein und Zeaxanthin; Extrakten von Serenoa repens; PDE5-Inhibitoren; Coenzym Q10; und Melatonin.

3. Zusammensetzung nach Anspruch 2, wobei die Menge an Vitamin D3 von 5 µg/ml bis 125 µg/ml und von 400IU bis 5000IU liegt.

4. Zusammensetzung nach einem der Ansprüche 1-3, ferner umfassend einen zweiten zusätzlichen Inhaltsstoff, ausgewählt aus der Liste von Acetylsalicylsäure, lipophilen Vitaminen, Polyphenolen, Flavonoiden, Bioflavonoiden, Coenzym Q10, Melatonin und Metformin, vorzugsweise ist dieser zweite zusätzliche Inhaltsstoff Acetylsalicylsäure.

5. Zusammensetzung nach Anspruch 4, wobei die Menge an Acetylsalicylsäure im Bereich von 0,05 pmol/ml bis 20 pmol/ml oder von 10 µg bis 650 µg liegt.

6. Verfahren zur Herstellung einer Nanoemulsion, umfassend die Zusammensetzung nach einem der Ansprüche 1-5, wobei das Verfahren mindestens einen Schritt, vorzugsweise 1-10 Schritte, bevorzugter 2-5 Schritte, zum Homogenisieren der Zusammensetzung von Rizinus- und Leinöl umfasst, wobei Leinöl vorzugsweise vor Rizinusöl in dem Verhältnis nach Anspruch 1 unter Kühlbedingungen für 1-5 Minuten, vorzugsweise für 1-2 Minuten, für einen jeden Homogenisierungsschritt zugesetzt wird, wobei die Homogenisierung bei 1000-5000 U/min, vorzugsweise bei 1500 U/min-3000 U/min, bevorzugter bei etwa 2000 U/min durchgeführt wird.

7. Verfahren nach Anspruch 6, wobei die Zusammensetzung ferner einen ersten zusätzlichen Inhaltsstoff nach einem der Ansprüche 2 oder 3 und/oder einen zweiten zusätzlichen Inhaltsstoff nach einem der Ansprüche 4 oder 5 umfasst.

8. Verfahren nach einem der Ansprüche 6 oder 7, wobei die Nanoemulsion, vorzugsweise bei Raumtemperatur, verfestigt wird, nachdem sie tropfenweise einer Lösung von Gelatine oder Maltodextrin zugesetzt wurde, wobei die Lösung von Gelatine oder Maltodextrin eine Konzentration von 10-30%, vorzugsweise etwa 20% aufweist oder die Nanoemulsion durch trockenes Erstarren oder Kieselgel verfestigt wird.

9. Nanoemulsion, die unter Verwendung des Verfahrens nach einem der Ansprüche 6-8 erhalten wurde.

10. Zusammensetzung nach einem der Ansprüche 1-5 oder Nanoemulsion nach Anspruch 9 zur Verwendung als Medikament, vorzugsweise als Antipyretikum oder Analgetikum.

11. Zusammensetzung nach einem der Ansprüche 1-5 oder Nanoemulsion nach Anspruch 9 zur Verwendung bei der Behandlung einer Krankheit, ausgewählt aus: Herzkrankheit, Magengeschwür, Leberschaden, Schlaganfall, Niereninsuffizienz, entzündlicher Krankheit, vorzugsweise ausgewählt aus: Spondylitis ankylosans, Osteoarthritis, rheumatoider Arthritis, systemischem Lupus erythematodes (SLE), SLE-assoziierter Arthritis und SLE-assoziierter Pleuritis oder rheumatischem Fieber, Myokardinfarkt und ischämischem Schlaganfall; und Malabsorptionssyndrom oder zur Verwendung bei der Prophylaxe von thromboembolischen Schlaganfall- oder Revaskularisierungsverfahren.

12. Zusammensetzungen nach Anspruch 4 oder 5 zur Verwendung bei der Behandlung von Nierenversagen, hämorrhagischen Läsionen, Magengeschwür, Hämostasekrankheit, Herz-Kreislauf-Schlaganfall, Hirnschlag, Leberinsuffizienz, iatrogener Erkrankung oder Reye-Syndrom.

13. Zusammensetzungen nach den Ansprüchen 1-5 zur Verwendung bei der Behandlung von Patienten, die sich einer Gerinnungshemmungstherapie, einer Antithrombosetherapie, einer postkardialen Operation oder einer posttraumatischen Therapie unterziehen.

14. Verwendung einer Zusammensetzung nach einem der Ansprüche 1-5 oder Nanoemulsion nach Anspruch 9 als Nutrazeutika, Nahrungsergänzungsmittel oder Supplementation.

## Revendications

1. Composition comestible comprenant de l'huile de ricin et de l'huile de lin, **caractérisée en ce que** le rapport volumétrique huile de ricin : huile de lin est de 25:75.

2. Composition selon la revendication 1 comprenant de plus au moins un premier ingrédient supplémentaire choisi dans la liste : vitamines lipophiles, de préférence choisies parmi : la vitamine A, la vitamine D, la vitamine E et la vitamine K, plus préférablement encore la vitamine D3 ; des poliphénols, de préférence choisis parmi : des anthocyanes, la curcumine, le resvératrol, la sylmarine et la quercétine ; des flavonoïdes ; des bioflavonoïdes ; des phytoestrogènes, de préférence une isoflavone, de préférence choisi parmi : la génistéine, la daidzéine et la glycitéine ; des caroténoïdes, de préférence choisis parmi : l'astaxanthine, le bêta-carotène, la crocétine, le lycopène, la lutéine et la zéaxanthine ; des extraits de chou palmiste ; des inhibiteurs PDE5 ; la coenzyme Q10 ; et la mélatonine.

3. Composition selon la revendication 2, dans laquelle la quantité de vitamine D3 est comprise entre 5 pg/ml et 125 pg/ml et entre 400 UI et 5 000 UI.

4. Composition selon l'une quelconque des revendications 1-3, comprenant de plus un second ingrédient supplémentaire choisi à partir de la liste comprenant l'acide acétylsalicylique, des vitamines lipophiles, des polyphénols, des flavonoïdes, des bioflavonoïdes, de la coenzyme Q10, de la mélatonine et de la metformine, de préférence ce second ingrédient supplémentaire étant l'acide acétylsalicylique.

5. Composition selon la revendication 4, dans laquelle la quantité d'acide acétylsalicylique est comprise entre 0,05 pmol/ml et 20 pmol/ml ou entre 10 µg et 650 µg.

6. Procédé de préparation d'une nanoémulsion comprenant la composition selon l'une quelconque des revendications 1-5, ledit procédé comprenant au moins une étape, de préférence 1-10 étapes, plus préférablement 2-5 étapes, d'homogénéisation de la composition des huiles de ricin et de lin, dans lequel l'huile de lin est de préférence ajoutée avant l'huile de ricin, selon le rapport selon la revendication 1 dans des conditions de refroidissement, pendant 1-5 minutes, de préférence pendant 1-2 minutes, pour chaque étape d'homogénéisation, dans lequel ladite homogénéisation est effectuée à 1 000-5 000 tours/min, de préférence à 1 500-3 000 tours/min, plus préférablement à environ 2 000 tours/min.

7. Procédé selon la revendication 6, dans lequel la composition comprend de plus un premier ingrédient supplémentaire selon l'une quelconque des revendications 2 ou 3 et/ou un second ingrédient supplémentaire selon l'une quelconque des revendications 4 ou 5.

8. Procédé selon l'une quelconque des revendications 6 ou 7, dans lequel la nanoémulsion est solidifiée, de préférence à température ambiante, après avoir été ajoutée en gouttes à une solution de gélatine ou de maltodextrine, ladite solution de gélatine ou de maltodextrine étant à une concentration de 10-30 %, de préférence d'environ 20 %, ou la nanoémulsion étant solidifiée par congélation sèche ou par un gel de silice.

9. Nanoémulsion obtenue en utilisant le procédé selon l'une quelconque des revendications 6-8.

10. Composition selon l'une quelconque des revendications 1-5, ou la nanoémulsion selon la revendication 9 pour une utilisation comme médicament, de préférence comme agent antipyrétique ou analgésique.

11. Composition selon l'une quelconque des revendications 1-5, ou la nanoémulsion selon la revendication 9 pour une utilisation dans le traitement d'une maladie choisie parmi : une maladie cardiaque, un ulcère de l'estomac, une lésion du foie, un AVC, une insuffisance rénale, une maladie inflammatoire de préférence choisie parmi : la spondylarthrite ankylosante, l'arthrose, la polyarthrite rhumatoïde, le lupus érythémateux disséminé (SLE), l'arthrite associée au SLE et la pleurésie associée au SLE, ou le rhumatisme articulaire aigu, l'infarctus du myocarde et l'accident vasculaire cérébral ischémique ; et le syndrome de malabsorption, ou pour une utilisation dans la prophylaxie d'un accident vasculaire cérébral thromboembolique ou les procédures de revascularisation.

12. Composition selon la revendication 4 ou 5 pour une utilisation dans le traitement de l'insuffisance rénale, des lésions hémorragiques, de l'ulcère gastrique, d'une maladie de l'hémostase, de l'accident cardiovasculaire, de l'AVC, de l'insuffisance hépatique, d'une maladie iatrogène ou du syndrome de Reye.

13. Compositions selon les revendications 1-5 pour une utilisation dans le traitement de patients suivant une thérapie anticoagulante, une thérapie anti-thrombose, une chirurgie post-cardiaque ou une thérapie post-traumatique.

14. Utilisation d'une composition selon l'une quelconque des revendications 1-5, ou de la nanoémulsion selon la revendication 9 comme nutraceutiques, complément nutritionnel ou complément alimentaire.
